# FASCICULE DE BREVET EUROPEEN

(11) **EP 3 380 180 B1**
(45) Date de publication et mention de la délivrance du brevet: **06.11.2019**
(21) Numéro de dépôt: 16815596.8
(22) Date de dépôt: 24.11.2016
(51) Int. Cl.: A61M 25/01, A61B 1/00, A61B 34/30

(54) **DISPOSITIF PNEUMATIQUE DE MAINTIEN ET DE DÉPLACEMENT D'UN OBJET ALLONGÉ ET SYSTÈME MÉDICAL INTÉGRANT UN TEL DISPOSITIF**
PNEUMATISCHE VORRICHTUNG ZUM HALTEN UND BEWEGEN EINES LÄNGLICHEN OBJEKTS UND MEDIZINISCHES SYSTEM MIT SOLCH EINER VORRICHTUNG
PNEUMATIC DEVICE FOR HOLDING AND MOVING AN ELONGATE OBJECT, AND MEDICAL SYSTEM INCORPORATING SUCH A DEVICE

(30) Priorité: 25.11.2015 FR 1561373
(43) Date de publication de la demande: 03.10.2018
(73) Titulaire: Université de Strasbourg (Etablissement Public National à Caractère Scientifique, Culturel et Professionnel), 67000 Strasbourg (FR); Centre National de la Recherche Scientifique (Établissement Publique National à Caractère Scientifique et Technologique), 75016 Paris (FR); Institut Hospitalo-Universitaire de Chirurgie Mini -Invasive Guidee Par l'Image de Strasbourg, 67000 Strasbourg (FR); Institut National des Sciences Appliquees (Etablissement Public à Caractère Scientifique, Culturel et Professionnel), 67000 Strasbourg (FR)
(72) Inventeur: NIERENBERGER, Mathieu, 67202 Wolfisheim (FR); BARBÉ, Laurent, 67730 Chatenois (FR); RENAUD, Pierre, Schiltigheim (FR); WACH, Benoît, 67000 Strasbourg (FR); BRUYAS, Arnaud, 42140 Saint Denis Sur Coise (FR)
(74) Mandataire: Nuss, Laurent
(86) Numéro de dépôt international: PCT/FR2016/053078
(87) Numéro de publication internationale: WO 2017/089711

(56) Documents cités:
- EP-A1- 2 875 792
- US-A1- 2015 297 203

## Description

La présente invention concerne le domaine de la manipulation assistée, voire automatisée, d'instruments ou d'outils, ou plus généralement de pièces ou d'objets allongés, en particulier dans des applications requérant une précision élevée et une sécurité maximale, tel que celui des interventions chirurgicales sur des sujets vivants ou des actions intervenant à proximité de l'homme, voire en interaction avec lui.

Dans ces contextes, l'invention a pour objet un dispositif pneumatique de maintien et de déplacement d'un objet allongé, un système médical ou industriel intégrant au moins un tel dispositif et un procédé d'insertion d'un objet allongé par l'intermédiaire d'un tel système.

Dans de nombreux domaines techniques, il existe un besoin pour des dispositifs automatiques ou semi-automatiques permettant de déplacer progressivement un élément ou une pièce allongé(e), en particulier en vue de l'introduire dans un corps ou un autre élément.

Cela est notamment vrai pour le domaine médical et plus spécifiquement la chirurgie.

Actuellement déjà, un nombre conséquent de dispositifs robotiques est proposé pour l'assistance aux gestes médicaux et chirurgicaux. Pour nombre d'entre eux, le système robotique est destiné à la manipulation d'outils tels que des instruments chirurgicaux, notamment sous forme de corps allongés, ou des aiguilles dans le cas par exemple de l'imagerie interventionnelle.

En chirurgie minimalement invasive, le passage par des trocarts conditionne la forme et la structure des outils et instruments utilisés et limite le nombre de mouvements indépendants réalisables. La translation selon l'axe des outils, de géométrie oblongue, est alors très sollicitée.

De la même manière, les procédures percutanées, qui consistent à insérer des aiguilles ou des outils médicaux analogues pour rejoindre une zone d'intérêt, nécessitent un mouvement de translation des aiguilles de large amplitude, qu'il y a lieu de réaliser avec un niveau de sécurité très élevée.

Dans tous ces contextes, si la géométrie oblongue et allongée des objets à déplacer est une constante, le diamètre de leur corps généralement cylindrique peut largement varier, et ceci même au cours d'un même geste. Il en est de même de leur longueur.

La plupart des solutions existantes se basent sur des systèmes d'actionnement solidaire de l'outil entraînant une modification de l'outil standard (ce qui n'est pas souhaitable). La translation est généralement réalisée par l'intermédiaire d'une transformation de mouvement (passage d'un mouvement rotatif à un mouvement de translation notamment). En cas de nécessité (panne, urgence médicale), le découplage entre l'outil et le système d'avance est assez complexe et peut engager la sécurité du patient. Le recours de plus en plus fréquent à des imageurs (échographie, fluoroscopie, scanner X, IRM) pendant la réalisation du geste rend par ailleurs délicat voire impossible l'emploi de nombre de technologies d'actionnement actuellement mises en oeuvre.

Différentes techniques connues de déplacement assisté, motorisé ou mécanisé, de type pas-à-pas ou séquentiel, appliquent les principes de la cinématique dite de la chenille arpenteuse ("inch-worm" en anglais), en particulier en relation avec des composantes gonflables ou actionnées pneumatiquement.

Différentes déclinaisons applicatives de ces principes ont déjà été proposées dans le domaine médical et chirurgical, soit pour permettre la progression au moins partiellement automatisée, d'un objet allongé (par exemple un endoscope) dans un conduit naturel d'un sujet (voir par exemple : EP 2 875 792, US 2010/022947 ; EP 1 792 561 ; US 5 398 670 ; WO 02/068035), soit pour déplacer, depuis l'extérieur et de manière partiellement automatisée, au moins un objet allongé devant être introduit dans le corps d'un sujet (voir par exemple : WO 2014/068563).

Toutefois, ces réalisations connues mettant en œuvre une cinématique du type "inch-worm" présentent au moins certains des inconvénients ci-après : structure complexe ; encombrement axial important ; composantes constitutives multiples ; incompatibilité avec certaines technologies d'imagerie médicale ; adaptation limitée à des objets présentant des diamètres variables ou de valeurs différentes ; utilisation de différents types d'énergie.

De plus, ces réalisations présentent toutes l'inconvénient commun, d'une part, de nécessiter plusieurs, c'est-à-dire au moins deux, lignes d'alimentation pour les différentes composantes actives des dispositifs de déplacement, d'autre part, de devoir piloter les différentes lignes de manière synchronisée (délicat à réaliser lorsque lesdites lignes ont des longueurs significatives) et, enfin de disposer d'une faculté limitée d'adaptation de l'engagement desdites composantes actives du dispositif avec l'objet allongé à déplacer.

Un autre domaine dans lequel il existe un besoin croissant de dispositifs, préférentiellement automatisés ou au moins semi-automatisés, est celui des contextes et environnements de travail dans lesquels un dispositif motorisé ou actionné doit fonctionner en interaction avec un opérateur humain, en imitant ou en assistant le geste d'un tel opérateur, ou encore les environnements à contraintes fortes, excluant notamment l'usage de technologies électromécaniques, électrotechniques ou électroniques.

Ainsi, on peut citer le cas des tâches d'assemblage de composants mécaniques sur des lignes d'assemblage, où des robots dits collaboratifs sont maintenant proposés pour aider l'opérateur. Pour ces dispositifs, la masse est un facteur de risque en cas de collision. L'existence d'une souplesse intrinsèque, que l'homme de métier désigne comme une compliance, est une propriété par ailleurs importante pour gérer le contact robot/opérateur. Enfin, il est nécessaire de proposer des robots de taille minimale pour ne pas gêner l'opérateur dans ses mouvements et sa collaboration avec le robot.

Dans le contexte industriel, on peut également citer les tâches automatisées de type dit "peg in hole" ("cheville dans trou" ou "d'enfichage") dans lesquelles des gestes d'assemblage de composants mécaniques sont réalisés. Les assemblages sont souvent basés sur des éléments de centrage pour référencer les positions des éléments. On peut citer le contexte de l'automobile par exemple avec l'assemblage d'éléments de moteur, ou l'insertion de connecteurs électriques dans l'industrie électrique et électronique. Il est connu qu'un maintien rigide des deux éléments à assembler ne permet pas la réussite d'un assemblage robotisé. Il est souvent nécessaire de disposer d'une compliance au moins limitée selon des directions perpendiculaires à l'axe d'insertion.

Par ailleurs, dans le contexte de la manipulation collaborative, la sécurité de l'opérateur humain est capitale. Pour assurer sa sécurité il faut non seulement contrôler la vitesse du système robotique mais également les efforts moteurs qu'il déploie.

Les technologies existantes du type dénommé "muscle artificiel" permettent de répondre au moins en partie aux problématiques et aux besoins exprimés ci-dessus.

Toutefois, elles présentent une grande complexité de réalisation et de commande, nécessitent des assemblages délicats et fastidieux et doivent impérativement être associés à des moyens de guidage cinématique additionnels.

Enfin, il existe également des contextes industriels où les contraintes d'environnement sont fortes et excluent l'usage de technologies électromécaniques. On peut citer le cas des ambiances explosives, du fait de la manipulation de produits intrinsèquement dangereux (matériaux explosifs) ou dans des conditions spécifiques comme par exemple la manipulation de poudres telles qu'utilisées dans des domaines comme la fabrication additive. Dans ce cas, l'emploi de technologies supprimant tout élément actif sur un plan électrique est un avantage certain.

La présente invention a pour but proposer une solution permettant de surmonter au moins certains des inconvénients et les principales limitations précités.

A cet effet, elle a pour objet un dispositif pneumatique de maintien et de déplacement d'un objet allongé, ledit dispositif comprenant un corps creux de forme générale annulaire ou tubulaire définissant une direction axiale, traversé par l'objet allongé au niveau d'un passage axial et constitué par deux parties ou segments d'extrémité arrangé(e)s de manière alignée dans la direction axiale et formant mors et un(e) partie ou segment médian(e) ou central(e) reliant les deux parties ou segments formant mors entre eux, avec faculté de déplacement relatif de l'un par rapport à l'autre dans la direction axiale sous l'effet d'une déformation contrôlée d'au moins une portion de ladite partie ou dudit segment médian(e) ou central(e),
dispositif caractérisé en ce que les différent(e)s parties ou segments du corps creux définissent des chambres respectives,
en ce que les partie(s) ou segment(s) d'extrémité formant mors comportent, en tant que portions de parois destinées à venir en contact avec l'objet allongé en phase de serrage, des membranes élastiquement déformables,
et en ce que les chambres des deux parties ou segments d'extrémité sont en communication fluidique avec la chambre de la partie ou du segment médian(e) ou central(e) par l'intermédiaire chacun(e) d'au moins un moyen calibré d'écoulement respectif, l'injection de fluide gazeux sous pression s'effectuant par le biais d'une unique ligne d'alimentation reliée à une source contrôlée de fluide sous pression et débouchant dans la chambre de l'un(e) des deux parties ou segments d'extrémité , ce en fonction du sens de déplacement en translation souhaité pour l'objet allongé présent dans le corps creux.

L'invention sera mieux comprise, grâce à la description ci-après, qui se rapporte à des modes de réalisation préférés, donnés à titre d'exemple non limitatif, et expliqués avec référence aux dessins schématiques annexés, dans lesquels :
la figure 1 illustre, sous forme d'une série de représentations schématiques, et de chronogrammes, les étapes successives de la cinématique développée par un dispositif de maintien et de déplacement selon l'invention pour réaliser un déplacement élémentaire (un pas séquentiel) d'un objet allongé, ce en relation avec l'évolution de la pression de fluide gazeux injecté par l'intermédiaire d'une unique ligne d'alimentation (rapport cyclique injection/évacuation 50/50) ;
la figure 2 est une vue en coupe d'un premier mode de réalisation pratique d'un dispositif selon l'invention, présentant une structure aplatie en direction axiale, de forme discoïdale ;
les figures 3A à 3G sont des vues en coupe similaires à celle de la figure 2, illustrant les différentes étapes de modification du dispositif de la figure 2 lors de la réalisation des étapes cinématiques d'un déplacement élémentaire (objet déplacé non représenté) ;
la figure 4A est une vue schématique en perspective et en coupe partielle selon un plan axial d'un second mode de réalisation de l'invention dans lequel la partie ou le segment médian(e) comporte un matériau à propriété auxétique ;
la figure 4B est une vue de détail, à une échelle différente, d'une formation élémentaire du motif formant la structure auxétique de la partie médiane représentée sur la figure 4A ;
les figures 5A et 5B sont des vues schématiques en perspective de la partie médiane d'une autre variante du second mode de réalisation du dispositif selon l'invention, intégrant un matériau auxétique à structure vrillée, et représenté respectivement en l'absence d'injection de fluide sous pression (au repos : figure 5A) et après atteinte d'une pression interne opérationnelle (figure 5B) ;
la figure 6 est une représentation schématique illustrant un exemple de mise en œuvre du dispositif selon l'invention dans un contexte médical ;
la figure 7 est un schéma synoptique d'un système d'alimentation contrôlée pour un dispositif selon l'invention, pouvant faire partie d'un système médical représenté figure 6 ;
la figure 8 représente des courbes de pression (en bar) en fonction du temps (en seconde) illustrant deux profils d'alimentation en air d'un dispositif selon l'invention avec des rapports cycliques injection/évacuation de 50/50 et de 70/30 ;
la figure 9 illustre, de manière similaire à la figure 1, les étapes successives de la cinématique développée par un dispositif selon l'invention alimenté avec un rapport cyclique 70/30 ;
les figures 10A à 10D sont des courbes illustrant respectivement : la course par cycle en fonction de la pression d'alimentation (figure 10A) ; la vitesse de déplacement de l'objet manipulé en fonction de la fréquence des cycles d'alimentation (figure 10B) ; les durées d'inflation et de déflation en fonction du diamètre des orifices (figure 10C) et la fréquence maximale des cycles en fonction du diamètre des orifices (figure 10D), ce pour un dispositif tel que représenté figures 2 et 3 ;
les figures 11A et 11B sont des représentations schématiques et synoptiques d'un dispositif selon deux variantes de réalisation de l'invention, dans lesquelles la chambre ou partie médiane ou centrale est déportée radialement ou latéralement par rapport aux parties ou chambres d'extrémité formant mors ;
la figure 12 est une représentation schématique en coupe et en élévation d'un dispositif selon un autre mode de réalisation de l'invention ;
la figure 13 illustre, sous la forme d'une série de représentations schématiques et de chronogrammes, les étapes successives de la cinématique développée par un dispositif tel que représenté figure 12 (mouvement de rotation et mouvement de translation, séquencés successifs) en relation avec l'évolution de la pression du fluide gazeux injecté ;
la figure 14 est une vue schématique en coupe et en élévation, similaire à celles des figures 1 et 12, d'un dispositif équipé de moyens de réglage des moyens calibrés d'écoulement (orifices traversants les parois communes aux chambres formant mors et à la chambre médiane ou centrale) ;
la figure 15 est une vue de dessus d'une paroi commune avec un orifice d'écoulement telle que représentée figure 14, le moyen de réglage étant représenté dans trois positions différentes (a, b, c) ;
la figure 16 est une vue similaire à celle des figures 5, le dispositif étant équipé d'un moyen capteur ;
les figures 17A à 17C sont des vues schématiques en coupe et en élévation d'une chambre ou partie d'extrémité formant mors d'un dispositif selon l'invention, illustrant différentes constructions particulières de la paroi ou de la membrane déformable interne de ladite partie (figure 17A : état desserré - figure 17B et 17C : état serré), et,
la figure 18 est une vue schématique en coupe et en élévation d'un dispositif selon l'invention tel que représenté figures 1, 12 et 13, équipé d'une électrovanne de commande de l'injection de fluide gazeux.

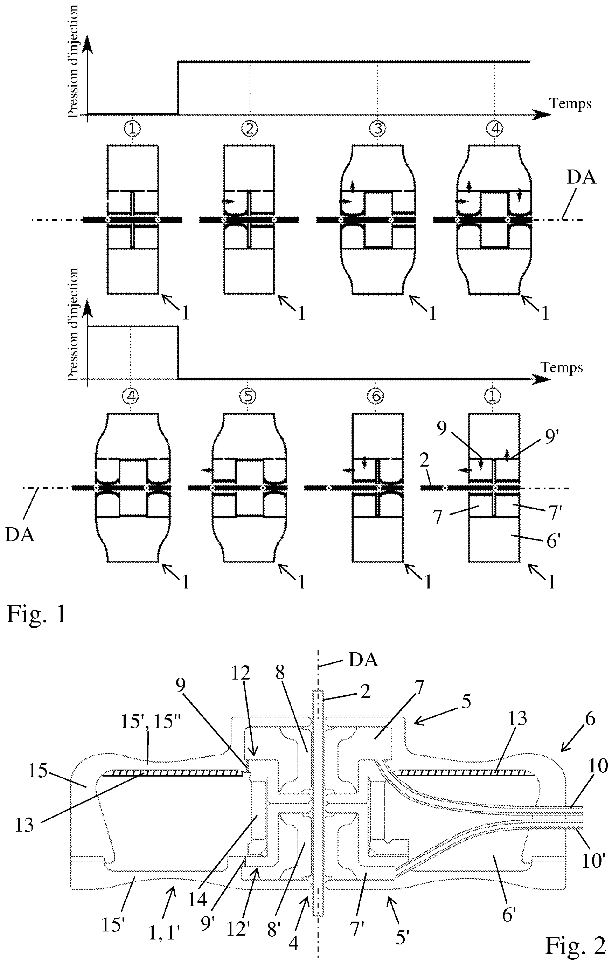

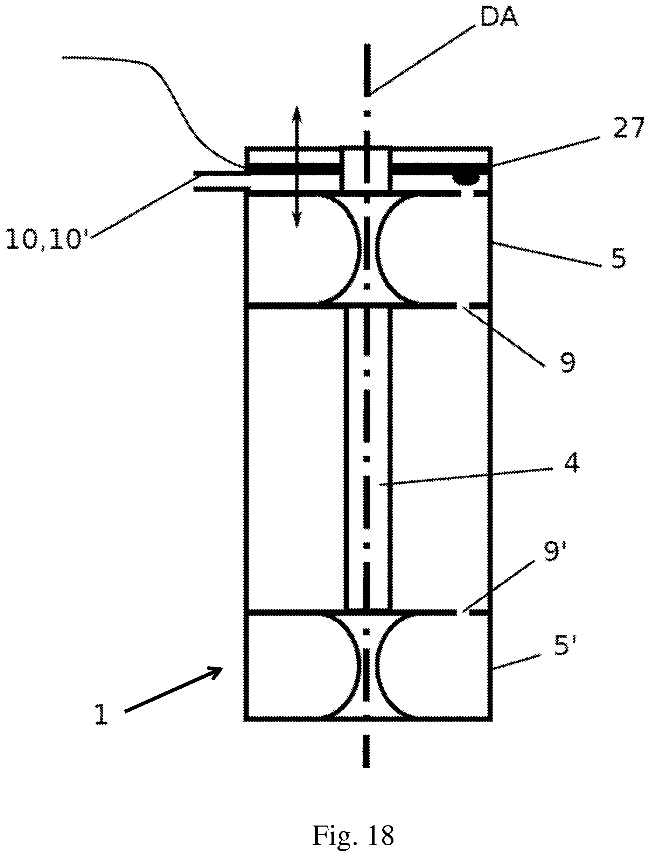

Les figures 1 à 4, 6, 9, 11 à 14, 16 et 18 illustrent toutes un dispositif pneumatique 1 de maintien et de déplacement automatique d'un objet allongé 2.

Ce dispositif 1 comprend un corps creux 1' de forme générale annulaire ou tubulaire définissant une direction axiale DA, traversé par l'objet allongé 2 au niveau d'un passage axial 4 et est constitué par deux parties ou segments d'extrémité 5 et 5' arrangé(e)s de manière alignée dans la direction axiale DA et formant mors et un(e) partie ou segment 6 médian(e) ou central(e) reliant les deux parties ou segments formant mors 5 et 5' entre eux, avec faculté de déplacement relatif de l'un par rapport à l'autre dans la direction axiale DA sous l'effet d'une déformation contrôlée d'au moins une portion 14, 15' de ladite partie ou dudit segment médian(e) ou central(e) 6.

Conformément à l'invention, il est prévu :
que les différent(e)s parties ou segments 5, 5', 6 du corps creux 1' définissent des chambres 7, 7', 6' respectives,
que les partie(s) ou segment(s) d'extrémité 5, 5' formant mors comportent, en tant que portions de parois 8, 8' destinées à venir en contact avec l'objet allongé 2 en phase de serrage, des membranes élastiquement déformables,
et que les chambres 7, 7' des deux parties ou segments d'extrémité 5, 5' sont en communication fluidique avec la chambre 6' de la partie ou du segment médian(e) ou central(e) 6 par l'intermédiaire chacun(e) d'au moins un moyen calibré d'écoulement 9, 9' respectif, l'injection de fluide gazeux sous pression s'effectuant par le biais d'une unique ligne d'alimentation 10, 10' reliée à une source contrôlée de fluide sous pression 11 et débouchant dans la chambre 7, 7' de l'un(e) 5 ou 5' des deux parties ou segments d'extrémité 5, 5', ce en fonction du sens de déplacement en translation souhaité pour l'objet allongé 2 présent dans le corps creux 1'.

Comme le montrent les figures 1 à 4, 6, 9, 12 à 14, 16 et 18, et en accord avec une première construction du dispositif 1, les parties ou segments d'extrémité 5, 5' et la partie ou le segment médian(e) ou central(e) 6 sont mutuellement alignées entre elles (eux) et toutes traversé(e)s par l'objet allongé 2, la direction axiale DA du corps creux 1' et l'axe de translation de l'objet allongé 2 étant confondus.

Les parties ou segments d'extrémité 5 et 5' présentent alors une disposition concentrique vu(e)s dans la direction DA.

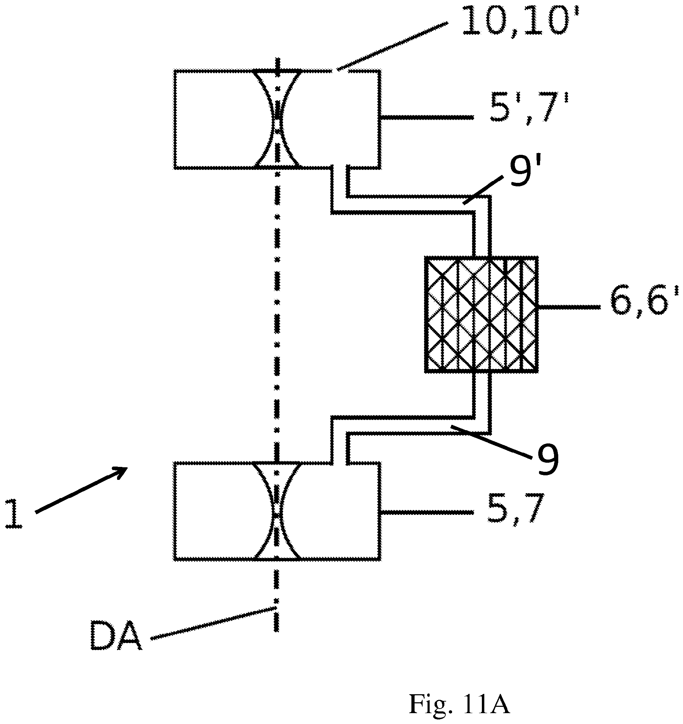

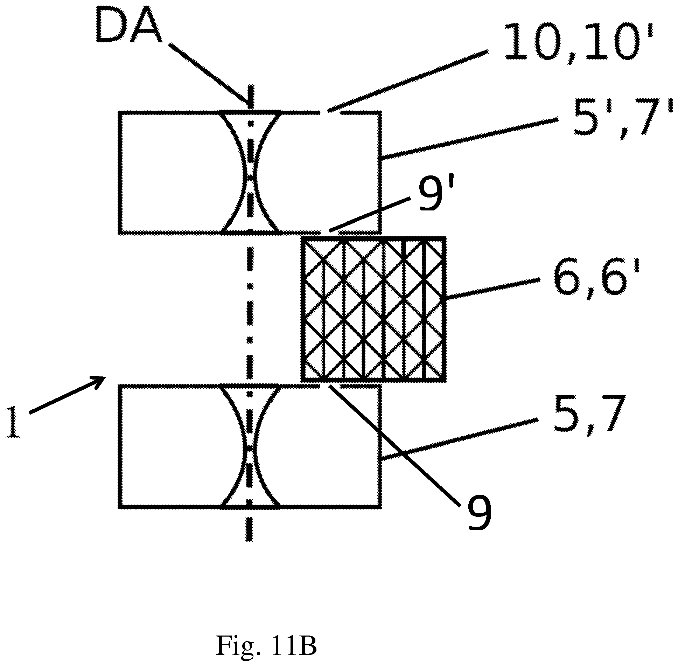

En accord avec une variante constructive de l'invention, illustrée schématiquement aux figures 11A et 11B, les parties ou segments d'extrémité 5, 5' sont mutuellement alignées entre elles (eux) et traversé(e)s tou(te)s deux par l'objet allongé 2, l'axe de translation de l'objet allongé 2 étant confondu avec l'axe d'alignement desdit(e)s parties ou segments d'extrémités 5, 5' correspondant à la direction axiale DA, la partie ou le segment médian(e) ou central(e) 6 étant décalé(e) latéralement ou radialement par rapport à cette direction DA et n'étant pas traversé par l'objet allongé 2.

La chambre médiane ou centrale 6' peut ou non partager des parois communes avec les chambres d'extrémités 7 et 7'.

Le fluide gazeux est préférentiellement de l'air sous faible pression (préférentiellement de 1 à 5 bars), éventuellement traité, aisément disponible dans les établissements de santé ou les sites industriels.

L'objet allongé 2 peut consister, par exemple :
- soit en un instrument ou outil chirurgical de forme oblongue et destiné à passer par un orifice ou un passage tubulaire,
- soit en une pièce, un outil ou un instrument destiné(e) à être introduit dans un orifice borgne ou traversant d'une seconde ou autre pièce.

Pour sa manipulation par le dispositif 1, l'objet 2 doit comporter au moins une partie allongée de forme cylindrique, s'il ne présente pas en totalité une constitution allongée cylindrique.

Ainsi, l'invention procure un dispositif 1 formant actionneur linéaire pour tout type de pièce, d'instrument ou d'outil 2 allongé(e), avec la possibilité (grâce au contact surfacique des membranes 8, 8') de saisir des pièces, outils ou instruments de tailles et de diamètres différents avec une prise ferme, sans que ces derniers n'aient à subir une quelconque modification (pour réaliser une éventuelle coopération, un hypothétique couplage ou emboîtement) et sans que leur surface extérieure ne soit modifiée ou dégradée par leur utilisation en relation avec le dispositif 1.

Le passage traversant axial 4 est avantageusement dimensionné en fonction des plus gros outils ou instruments 2 prévus pour une manipulation par le dispositif 1 (objets ou pièces de plus gros diamètre).

Le déplacement n'étant pas réalisé avec un mouvement continu, mais de manière séquentielle, les risques de déplacement non contrôlé, voire de déplacement non souhaité, sont limités (en fréquence et en ampleur), voire éliminés. La sécurité du geste, dans un contexte médical, est donc fortement augmentée. De plus, compte tenu des forces en jeu et de la vitesse d'exécution des mouvements élémentaires successifs, une intervention humaine dominante et correctrice est toujours possible.

En outre, compte tenu de la structure enveloppante du corps creux, ou au moins des deux mors d'extrémité 5 et 5', autour de l'objet oblong 2 à déplacer, un maintien de ce dernier dans un tube de diamètre déterminé est toujours garanti, tout en autorisant une liberté de mouvement encadrée dans ce volume intérieur cylindrique (passage axial 4). En effet, à l'état dégonflé ou au repos, le dispositif 1, avec son passage tubulaire 4 dans lequel est disposé l'outil ou instrument 2, évite un débattement trop important par rapport à la direction axiale DA, mais autorise un mouvement suffisant permettant, par exemple, à une aiguille chirurgicale plantée dans un organe de suivre le mouvement physiologique de ce dernier (mouvement de rotation de l'aiguille par rapport à son point d'insertion dans le corps du sujet 19) ou encore d'être manipulée par le praticien.

Par ailleurs, et en accord avec un avantage important procuré par l'invention, l'alimentation par une seule ligne 10, 10' (par sens de déplacement) simplifie grandement la connectique, réduit l'encombrement au niveau du site de mise en œuvre du dispositif 1 et facilite la gestion du fluide. En effet, les différentes étapes cinématiques consécutives, nécessaires pour aboutir au déplacement de l'objet 2 par pas élémentaires successifs, sont effectuées naturellement et sans intervention active autre que l'injection et l'arrêt de l'injection de fluide sous pression, du fait de la mise en communication calibrée particulière des trois chambres 7, 6' et 7' qui détermine l'ordre, la durée et l'intensité des séquences d'activation (mise sous pression) et de désactivation (mise hors pression) de ces dernières générant un déplacement du type "inch-worm".

Enfin, compte tenu de sa nature pneumatique et constructivement déformable, le dispositif 1 présente dans toutes les circonstances de mise en œuvre (même gonflé) une compliance axiale et/ou radiale au moins minimale.

Avantageusement, les parties ou segments d'extrémité 5, 5' formant mors pneumatiques, respectivement frontal et arrière en fonction du sens de déplacement positif de l'objet allongé 2, délimitent des chambres 7, 7' de forme annulaire ou torique de révolution autour de la direction axiale AD, et présentant une structure sensiblement indéformable, axialement et radialement, dans la gamme de pressions opérationnelles de fluide injecté en fonctionnement normal du dispositif 1, à l'exception de membranes élastiquement déformables formant des manchons 8, 8' internes constituant les logements traversant des mors pour l'objet allongé 2, formant par alignement le passage axial 4 du corps creux 1' multichambre.

Ainsi, le dispositif 1 comprend deux chambres annulaires ou toriques avec une paroi 8 ou 8' déformable élastiquement, sous pression interne, radialement vers l'intérieur (fonction mors pneumatiques) et une chambre annulaire ou tubulaire 6' médiane de liaison, déformable élastiquement sous pression interne par allongement ou extension axiale du segment médian creux 6 qui la délimite.

Préférentiellement, la partie ou le segment médian(e) ou central(e) 6 présente au moins une portion de paroi commune 12, 12' avec respectivement chacun(e) des deux parties ou segments d'extrémité 5, 5', chacune desdites portions de parois communes 12, 12' participant ainsi simultanément à la délimitation partielle de l'une des chambres d'extrémité 7, 7' et à celle de la chambre médiane ou centrale 6'. Dans ce cas, les moyens d'écoulement 9, 9' calibrés peuvent consister en des trous traversants ménagés dans lesdites portions de parois communes 12, 12' et présentant des formes et des tailles déterminées, fonction du séquencement souhaité pour le gonflement et le dégonflage des chambres 7, 6', 7' déterminant l'amplitude et/ou la vitesse du mouvement de déplacement en translation de l'objet allongé 2 considéré.

En accord avec une caractéristique de l'invention, le calibrage de chacun des moyens d'écoulement respectifs 9, 9' entre chacune des chambres d'extrémité 5, 5' et la chambre médiane ou centrale 6, qui est éventuellement différent et propre pour chaque moyen 9, 9', est réglé de telle manière que, par contrôle passif de la circulation du fluide gazeux injecté par l'unique ligne d'alimentation 10, 10', les deux chambres d'extrémité 5 et 5' peuvent être sous une pression opérationnelle entraînant un double serrage effectif de l'objet 2 à déplacer, lorsque la chambre médiane ou centrale 6 est sous pression opérationnelle et dans son état déformé.

L'homme du métier comprend que le contrôle passif de l'écoulement du fluide gazeux entre les différentes chambres 6', 7, 7' communicantes, autant durant les phases d'injection (gonflement) que durant les phases d'évacuation (dégonflement), dépend prioritairement du dimensionnement des moyens 9 et 9', à savoir le diamètre de chacun des trous 9 et 9' dans le cadre de la variante de réalisation préférée de l'invention. Ces écoulements contrôlés déterminés par dimensionnement à la fabrication, définissent les durées des séquences de gonflement et de dégonflement des chambres 6', 7, 7' durant les deux phases, en relation bien entendu avec les volumes desdites chambres et leur coefficient d'expansion par déformation, avec les pressions/dépressions appliquées, avec la nature du fluide gazeux utilisé (normalement de l'air) et avec le rapport cyclique mutuel des phases de gonflement et de dégonflement.

Un autre paramètre d'importance est la force de serrage souhaitée au niveau de chaque mors 5, 5'.

Pour un dispositif 1 tel que représenté figure 2, avec des membranes 8, 8' ayant des longueurs axiales d'environ 10 mm chacune et des chambres 6, 7, 7' ayant des volumes de 10⁻³ à 10⁻⁴ m³ et alimentées en air sous une pression d'environ 2 bars, chacun des deux trous 9, 9' présente avantageusement un diamètre d'environ 1 mm, permettant d'aboutir à une force de serrage d'environ 8 à 10 N avec une fréquence cyclique d'environ 1 Hz et un pas cyclique d'environ 1 mm, selon les expériences et essais réalisés par les inventeurs.

Une simulation basée sur les lois et règles de la mécanique des fluides et de la déformation élastiques des corps, aisément à la portée de l'homme du métier, peut permettre de dimensionner le dispositif 1 et ses paramètres de fonctionnement en fonction du résultat opérationnel recherché.

Les étapes chronologiquement successives d'un cycle cinématique du dispositif 1 tel qu'il est représenté aux figures 2 et 11 par exemples, aboutissant à un déplacement d'un pas élémentaire de l'objet 2 sont illustrées sur la figure 1 (en grisé : chambres sous pression opérationnelle ou au moins sous une pression déformante) :
- étape 1 (en double sur la figure 1 : début et fin de cycle) : le dispositif 1 est au repos et le corps creux 1' n'est soumis à aucune pression gazeuse (état dégonflé - à pression atmosphérique) :
   l'objet 2 n'est pas maintenu rigidement et n'est pas solidaire du corps creux 1', il peut être manipulé directement par l'utilisateur (débattement limité).
- étape 2 : début d'un cycle de déplacement en translation : de l'air comprimé est injecté par la ligne d'alimentation 10 dans la chambre 7 de la partie d'extrémité 5 frontale et la membrane tubulaire 8 de ce mors vient en application interne (avec une pression prédéterminée) contre la surface locale en regard de l'objet 2 (saisie de ce dernier).
- étape 3 : lorsque le premier mors 5 est sensiblement gonflé à sa pression opérationnelle (donc après écoulement d'un certain délai), l'air s'échappe par au moins un orifice calibré 9 dans la chambre 6' de la partie médiane 6, qui s'allonge dans la direction axiale DA sous la pression d'air et sépare les deux mors 5 et 5'.
- étape 4 (illustrée en double sur la figure 1) : lorsque la chambre 6' médiane est gonflée à sa pression opérationnelle, c'est-à-dire lorsque la partie médiane 6 a atteint son degré d'élongation nominal, l'air s'échappe par au moins un orifice calibré 9' vers la chambre 7' de la partie d'extrémité arrière 5' et la membrane tubulaire 8' de ce mors vient en application intime (avec une pression prédéterminée) contre la surface locale de l'objet 2 en regard (contact surfacique local) lorsque la pression opérationnelle est atteinte dans la chambre 7' (double saisie de l'objet : maintien par les deux mors 5 et 5' avec un écartement défini par l'élongation de la partie médiane 6).
- étape 5 : l'alimentation en air comprimé est stoppée et l'air sous pression est évacué à travers la ligne d'alimentation 10 faisant office de ligne d'évacuation. La chambre 7 de la partie d'extrémité frontale 5 se vide et le mors correspondant libère sa prise sur l'objet 2.
- étape 6 : l'air sous pression contenu dans la chambre 6' de la partie médiane 6 s'évacue et cette partie 6 reprend sa forme au repos (non allongée). Les deux mors 5 et 5' se rapprochent mutuellement et le mors arrière 5', toujours en prise avec l'objet 2, le déplace à travers le mors frontal 5 (maintenu fixe).
- étape 7 : finalement la chambre 7' de la partie d'extrémité arrière 5' se vide également et le mors correspondant relâche l'objet 2, qui se retrouve libre dans le passage axial tubulaire 4 (on retrouve une situation identique à celle de l'étape 1).

Le cycle précité décrit en relation avec les représentations de la figure 1 repose sur un rapport cyclique de valeur 1 entre les phases d'injection et d'évacuation du fluide (pour chaque cycle correspondant à un déplacement élémentaire de l'objet 2, le temps de chacune des deux phases représentent 50 % de la durée du cycle) et comporte une étape transitoire de passage entre deux cycles consécutifs lors de laquelle l'objet 2 est relâché (étapes 1 et 7 de la figure 1).

Lorsque, par exemple dans le cas d'un enfoncement avec retour élastique ou résistance à l'enfoncement, on veut conserver une prise continue durant tout le mouvement d'enfoncement, le rapport cyclique entre les deux phases peut être modifié (par exemple 70/30) de telle manière que le gonflement du mors 5 frontal débute avant que le dégonflement du mors 5' arrière ne soit achevé, voire n'ait débuté. Dans ce cas, le dispositif 1 est en prise permanente avec l'objet 2 durant tout le déplacement par pas cyclique de ce dernier.

Afin d'amplifier le phénomène d'allongement de la partie médiane ou centrale 6, et par conséquent le pas de déplacement par cycle cinématique, il peut être prévu que cette partie 6 soit au moins partiellement formé(e) ou recouvert(e) d'une couche 13 d'un matériau auxétique, c'est-à-dire avec un coefficient de Poisson négatif. Le caractère auxétique dudit matériau peut provenir de la structure de ce dernier et/ou de la nature même dudit matériau.

Conformément à une variante de réalisation pratique préférée, le corps creux 1' multichambre avec ses trois sorties ou segments 5, 5' est entièrement réalisé en matériau(x) polymère(s), préférentiellement en tant que pièce monobloc ou réalisée d'un seul tenant, par exemple par impression tridimensionnelle, les membranes déformables 8, 8' des mors 5, 5' étant éventuellement rapportées par surmoulage d'un matériau polymère distinct spécifique.

Ainsi, le dispositif 1 peut être mis en œuvre dans un contexte quelconque d'imagerie médicale et être fabriqué pour un coût de revient faible, éventuellement sous forme de produit à usage unique.

De manière avantageuse néanmoins, les matériaux polymères utilisés, par exemple de la résine acrylate rigide (de nature élasto-plastique) pour les parois rigides du corps creux 1' et de la résine acrylate souple (de nature élastomérique) pour les parois élastiquement déformables de ce dernier, sont de nature à résister à une stérilisation et compatible avec une utilisation en chambre stérile.

En variante, le matériau rigide peut être un polypropylène ou un polyoxyméthylène et le matériau souple un silicone.

Les parois du corps creux 1' peuvent également être réalisées en un alliage à mémoire de forme, avec éventuellement une configuration spécifique desdites parois, pour une déformation contrôlée et réversible en fonction de la pression interne dans les chambres 6', 7, 7'. Un matériau élastiquement souple peut être rapporté par surmoulage pour former les membranes 8, 8'.

En vue de disposer d'un dispositif 1 apte à déplacer l'objet allongé 2 dans les deux sens de la direction axiale AD, sans modifier leur arrangement mutuel, et donc pouvoir enchaîner des mouvements d'introduction et d'extraction, il peut être prévu qu'un conduit 10, 10' apte et destiné à former sélectivement une ligne d'alimentation ou une ligne d'évacuation est relié à chacune des chambres 7, 7' des parties ou segments d'extrémité 5, 5' (figure 2).

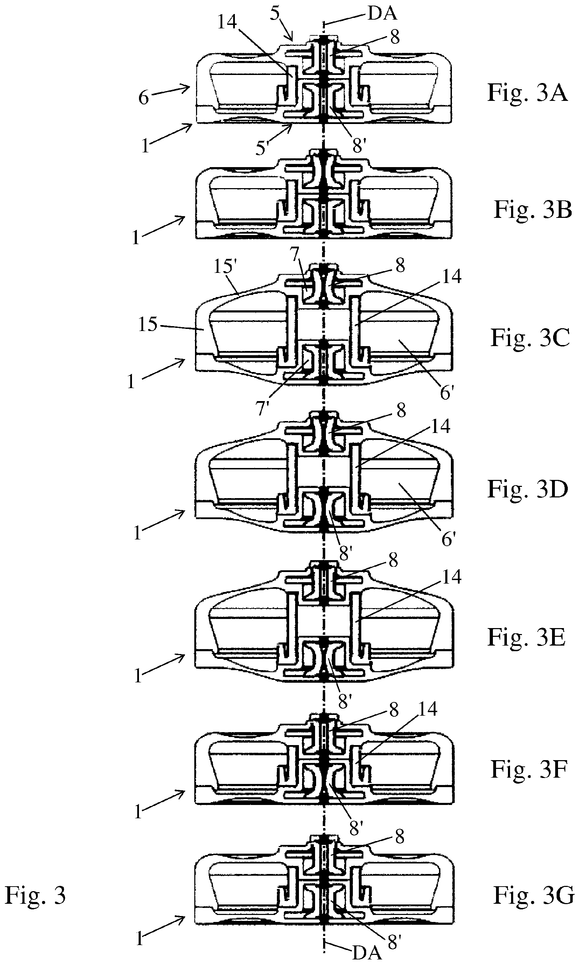

Selon un premier mode de réalisation de l'invention, illustré aux figures 2 et 3, la partie ou le segment médian(e) ou central(e) (6) définit une chambre 6' annulaire ou torique et s'étend périphériquement, et radialement extérieurement, autour des deux parties ou segments d'extrémité 5, 5' pour aboutir à un corps creux 1' multichambre de forme discoïdale. Cette partie ou ce segment 6 relie ces parties ou segments 5, 5' entre eux/elles par une liaison élastiquement déformable dans la direction axiale DA, les deux parties ou segments d'extrémité 5, 5' étant en contact mutuel dans ladite direction axiale DA à l'état dégonflé de la partie ou du segment médian(e) ou central(e) 6 et les deux parties ou segments d'extrémité 5, 5' étant situé(e)s à distance l'un(e) de l'autre à l'état gonflé de ladite partie ou dudit segment médian(e) ou central(e) 6, sous une pression suffisante pour entraîner une déformation de ce(tte) dernier(e). La déformation de ce(tte) dernier(e) entraînant un écartement nominal des mors gonflables 5 et 5' est atteinte lorsque la valeur de pression opérationnelle est atteinte dans la chambre médiane ou centrale 6'.

Dans ce mode de réalisation, le dispositif 1 présente au repos une extension longitudinale selon la direction DA de valeur minimale, pour une surface de contact des mors 5, 5' avec l'objet 2 qui est maximale.

En effet, la partie médiane 6 et sa chambre associée 6' s'étendent autour des mors 5 et 5' et présente une dimension axiale qui est inférieure au cumul des dimensions axiales des deux mors 5, et 5'. Ainsi, au repos, la dimension axiale du dispositif 1 correspond à la somme des dimensions axiales des deux mors 5 et 5', qui se retrouvent axialement en contact.

Conformément à une construction pratique avantageuse en relation avec le premier mode de réalisation précité, la partie ou le segment médian(e) ou central(e) 6 du corps creux 1' multichambre est constitué(e), d'une part, par une portion de paroi tubulaire interne 14 pouvant être déformée élastiquement dans la direction axiale DA et reliant les deux parties ou segments d'extrémité 5, 5' entre eux(elles) et, d'autre part, par une portion de paroi externe à section en U 15, de révolution autour de la direction axiale DA et formant par coopération complémentaire avec la portion de paroi tubulaire interne 14, la chambre médiane ou centrale 6' annulaire ou torique du corps creux 1', les deux ailes 15' du U étant également reliées par leurs extrémités libres aux deux parties ou segments d'extrémité 5, 5' et étant déformables élastiquement par flexion vers l'extérieur du U dans la direction axiale DA, sous l'effet d'une surpression suffisante dans la chambre centrale ou médiane 6'.

Le gonflement de la chambre médiane ou centrale 6' entraîne par conséquent un écartement élastique des ailes 15' de la structure de révolution à section en U 15 et simultanément un étirement élastique de la paroi tubulaire interne 14 dans la direction axiale DA, aboutissant à un écartement des deux parties ou segments d'extrémité 5 et 5' entre eux.

En fonction de la cinématique souhaitée, l'une des deux ailes 15' (par exemple l'aile 15' frontale associée à la partie d'extrémité 5) peut présenter une plus grande aptitude à la déformation, voire être la seule aile déformable. Une déformation élastique simultanée des ailes 15' frontale et arrière permet d'augmenter le pas cyclique du dispositif 1 (voir figures 3E et 3F).

La partie ou le segment médian(e) ou central(e) 6, en plus de définir une chambre centrale ou médiane 6' se dilatant sous la pression, réalise une double liaison élastique entre les deux parties ou segments d'extrémité 5 et 5'.

Les orifices 9 et 9' de communication fluidique sont ménagés quant à eux dans les portions de paroi 12 et 12' situées entre les ailes 15' et les sites de solidarisation de la paroi tubulaire 14 sur les parties ou segments d'extrémité 5 et 5', au niveau des parois rigides de ces dernier(e)s formant avec les membranes 8 et 8' les chambres annulaires 7 et 7'.

En vue d'aboutir éventuellement à un déplacement plus important à chaque cycle, les zones 15" de la portion de paroi à section en U externe 15 qui correspondent aux ailes 15' dudit U sont formées ou recouvertes, préférentiellement sur leur face interne, par une couche 13 de matériau auxétique (figure 2) ou à propriété auxétique.

A titre d'exemple pratique de construction d'un dispositif 1 réalisé par les inventeurs, sur la base du mode de réalisation représenté figures 2 et 3, les dimensions et coefficients suivants peuvent être appliqués :
- Dimensions extérieures du dispositif 1 :
   - diamètre extérieur : 74 mm
   - épaisseurs extérieures : 24 mm
- Mors 5 et 5' :
   - volume au repos : 2,1 x 10⁻⁶ m³
   - diamètre intérieur repos : 2,0 x 10⁻³ m
   - diamètre aiguille : 1,8 x 10⁻³ m
   - longueur membranes 8, 8' : 10,0 x 10⁻³ m
   - pression avant contact : 0,5 x 10⁵ Pa
- Chambre médiane 6 :
   - volume au repos : 4,2 x 10⁻⁵ m³
   - coefficient de raideur volumique : 1,3 x 10⁻¹¹ m³/Pa
   - coefficient de raideur en déplacement : 4,5 x 10⁻⁹ m/Pa
- Trous 9 et 9' :
   - diamètre : 1,0 x 10⁻³ m
   - coefficient de décharge : 0,577

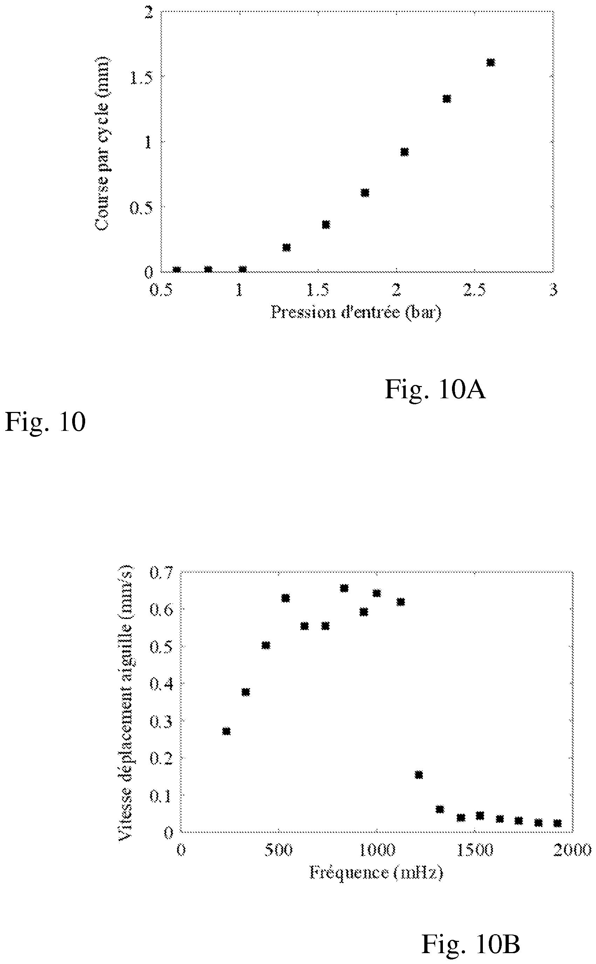

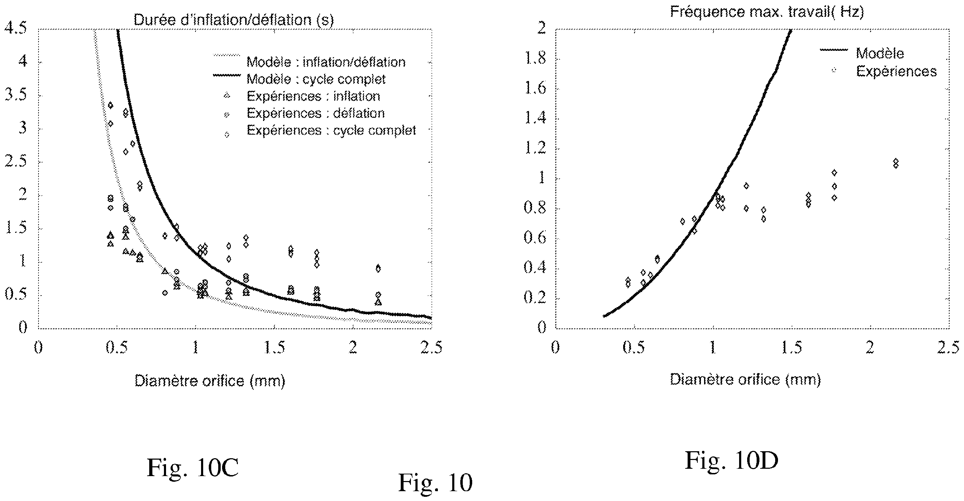

En construisant et en utilisant un dispositif 1 dimensionné comme indiqué ci-dessus et en modélisant son comportement, les inventeurs ont pu établir les courbes des figures 10A à 10D, desquelles ressortent les valeurs typiques, limites et optimales en termes de dimensionnement des orifices 9, 9' de communication entre chambres, de pression d'injection, de fréquence cyclique et de pas de cycle. A partir de ces valeurs, il est évident pour l'homme du métier de décliner des variantes dimensionnelles du dispositif 1.

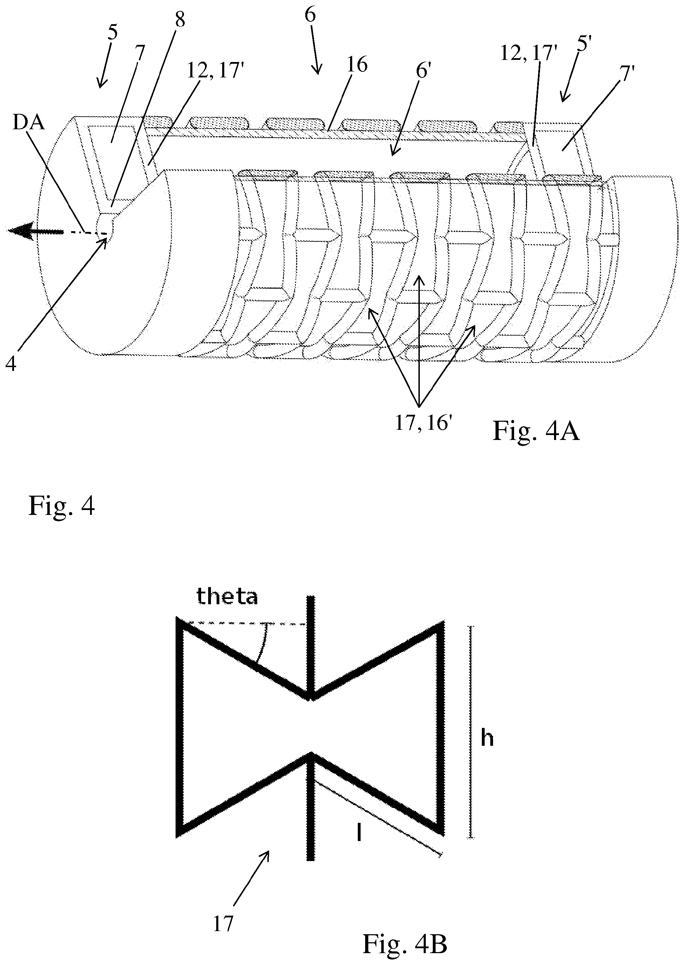

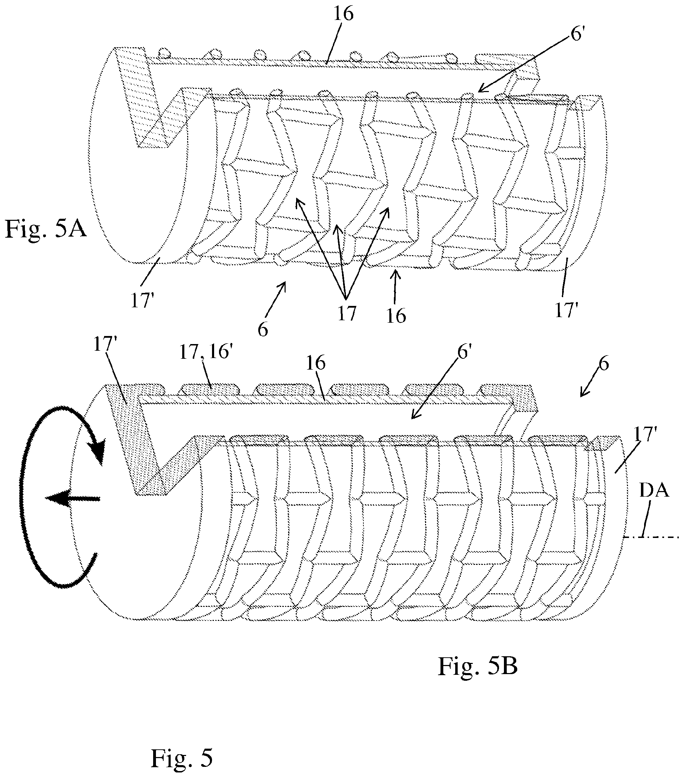

Selon un second mode de réalisation de l'invention, ressortant des figures 4 et 5, la partie ou le segment central(e) ou médian(e) 6 peut comprendre une chambre tubulaire cylindrique 6' s'étendant dans la direction axiale DA et de révolution autour de cette dernière, ladite chambre 6' comportant une paroi extérieure 16 déformable élastiquement et étant en communication fluidique calibrée avec les chambres 7, 7' des parties ou segments d'extrémité 5, 5'. Ladite paroi 16 est recouverte extérieurement d'une structure cylindrique à propriété auxétique 16', qui est formée d'un réseau maillé à motif répétitif de formations élémentaires 17 et qui prend appui sur et est solidaire de deux butées discoïdales 17' opposées. Ces butées 17' sont également reliées à la paroi déformable 16 précitée et forment des portions de paroi communes 12, 12' avec aux parties ou segments d'extrémité 5, 5' ou sont reliées à ces dernières. Les composantes constitutives précitées de la partie ou du segment médian(e) 6 sont mutuellement arrangés de telle manière que la mise sous pression opérationnelle de la chambre médiane ou centrale 6' entraîne par action de la paroi 16 sur le réseau 16' un écartement mutuel des butées discoïdales 17' dans la direction axiale DA, et donc des parties ou segments d'extrémité 5, 5' respectivement solidaires desdites butées 17'.

Conformément à une construction pratique avantageuse, les formations élémentaires 17 constituent ensemble un réseau 16' à motif en nids d'abeilles inversés et sont arrangées en plusieurs rangées consécutives dans la direction DA.

Lorsqu'un mouvement plus complexe qu'une simple translation est recherché, il peut par exemple être prévu que le réseau auxétique 16' présente au repos un vrillage autour de la direction axiale DA, une mise sous pression opérationnelle dudit réseau entraînant un déplacement relatif en translation et un déplacement relatif en rotation entre les deux parties ou segments d'extrémité 5 et 5' (figures 5A et 5B, par opposition à la variante des figures 4A et 4B dont le réseau 16' n'est pas vrillé).

La figure 4B montre les paramètres dimensionnels essentiels des formations élémentaires 17, qui définissent la géométrie et les performances de déformations du réseau auxétique 16'. A titre d'exemple illustratif, et en choisissant théta = 10 degrés, l = 11 mm et h = 6,8 mm, un réseau 16' comportant des bandes de quatre formations élémentaires 17 consécutives sur la circonférence et dix telles bandes de formations 17 répétées sur la longueur axiale du réseau 16' permet de réaliser des déplacements cycliques de 3 mm environ sous une pression d'environ 1 bar.

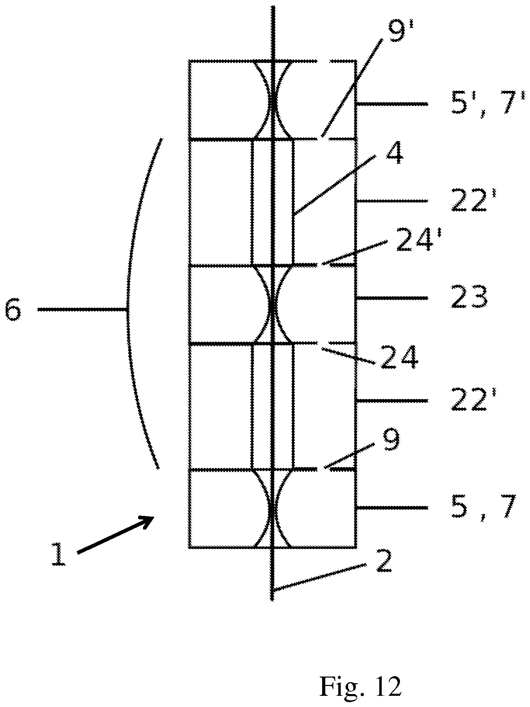

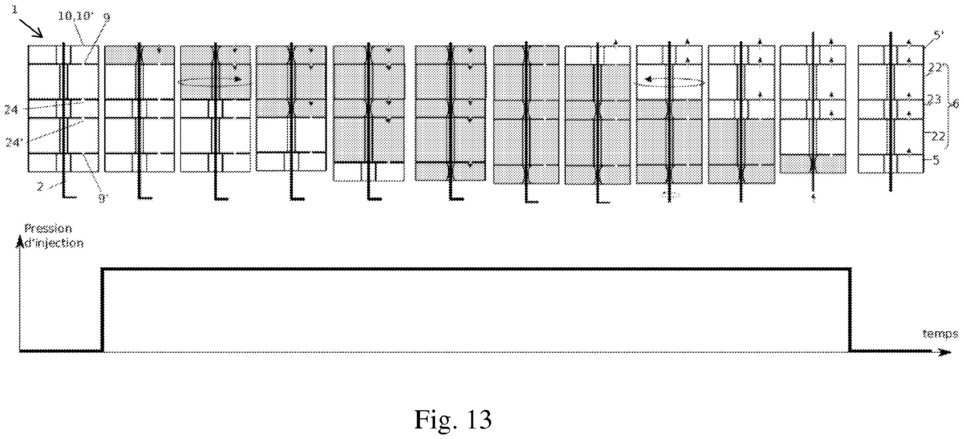

En accord avec une autre variante constructive de l'invention, illustrée aux figures 12 et 13 et autorisant un mouvement séquentiel de l'objet 2, la partie ou le segment central (e) 6 peut comprendre trois chambres ou parties fonctionnelles contiguës 22, 22', 23, à savoir, d'une part, deux chambres 22, 22' aptes et destinées à assurer une fonction de déplacement sous l'effet d'une déformation d'au moins une portion de leur paroi résultant d'une modification de leur pression interne, et accolées chacune respectivement à l'un(e) des parties ou segments d'extrémité 5, 5' formant mors gonflables, avec lesquel(le)s ils (elles) sont respectivement en communication fluidique par un moyen calibré d'écoulement 9, 9', et, d'autre part, une partie ou chambre fonctionnelle médiane 23 formant mors, située entre les deux chambres ou parties fonctionnelles 22 et 22' précitées et en communication fluidique par un moyen calibré d'écoulement respectif 24, 24' avec chacune d'entre elles, l'ensemble des cinq parties 5, 5', 22, 22',23 formant une structure cylindrique.

Dans le cadre de cette variante constructive, il peut être prévu qu'une première des deux chambres fonctionnelles 22 est configurée pour assurer un déplacement en rotation autour de la direction axiale AD de l'élément allongé 2 serré par un 5 des deux mors d'extrémité 5, 5' et que la seconde des deux chambres fonctionnelles 22' est configurée pour assurer un déplacement en translation le long de la direction axiale AD de l'objet allonge" 2 serré par le mors médian 23 et l'autre mors d'extrémité 5'.

Le mouvement séquencé résultant est représenté sur la figure 13 à titre d'exemple.

Un tel dispositif 1 double correspond fonctionnellement et constructivement à l'agglomération de deux dispositifs 1 accolés axialement (un pour la rotation et un pour la translation), le mors central 23 étant partagé et commun aux deux.

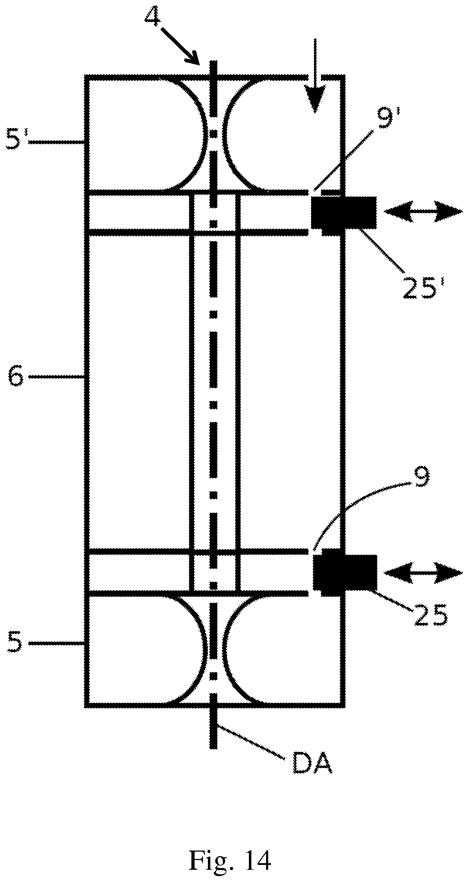

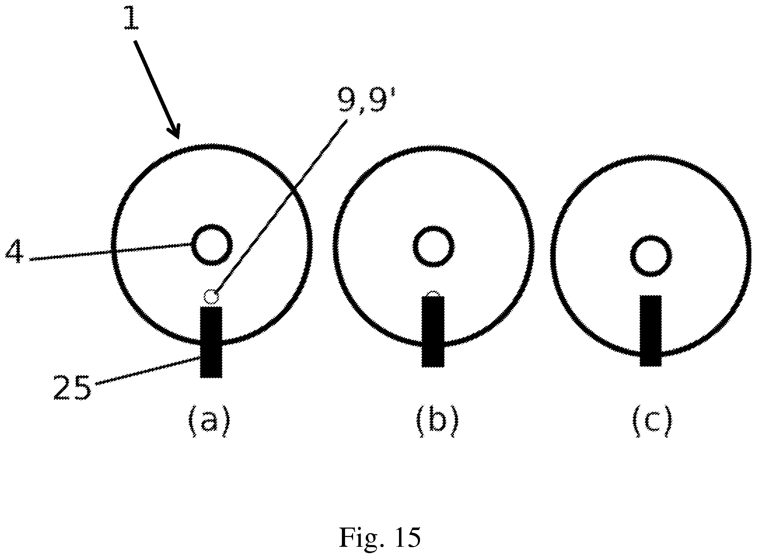

Afin de pouvoir faire varier notamment les durées de cycle et/ou les vitesses de certaines phases optatives, le dispositif 1 peut comprendre, comme le montrent les figures 14 et 15, au moins un moyen de réglage 25 d'au moins un moyen calibré d'écoulement 9, 9', 24, 24', en particulier de sa section de passage, un tel moyen de réglage 25 étant préférentiellement associé à chaque moyen d'écoulement 9, 9', 24, 24'.

Avantageusement, le ou chaque moyen de réglage 25, à actionnement manuel ou motorisé, comprend un élément mobile d'obturation variable de l'orifice calibrée 9, 9', 24, 24' formant le moyen d'écoulement considéré.

Le réglage précité peut se faire en début de cycle et être constant durant le cycle ou durant deux phases d'un même cycle.

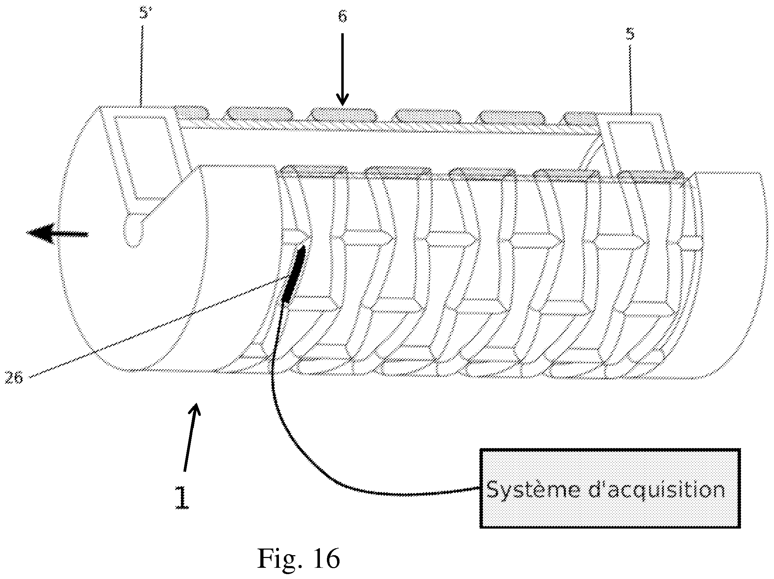

Comme représenté schématiquement sur la figure 16, le dispositif 1 peut aussi comprendre un moyen capteur 26, rapporté ou intégré à la partie ou au segment médian(e) ou central(e) 6 et apte et destiné à délivrer un signal fonction de l'élongation de cette partie ou de ce segment dans la direction axiale AD et/ou autour de cette dernière.

Ce moyen 26 est relié à un système d'acquisition et les informations fournies sont par exemple utilisées par le système de commande du dispositif 1 pour contrôler son fonctionnement.

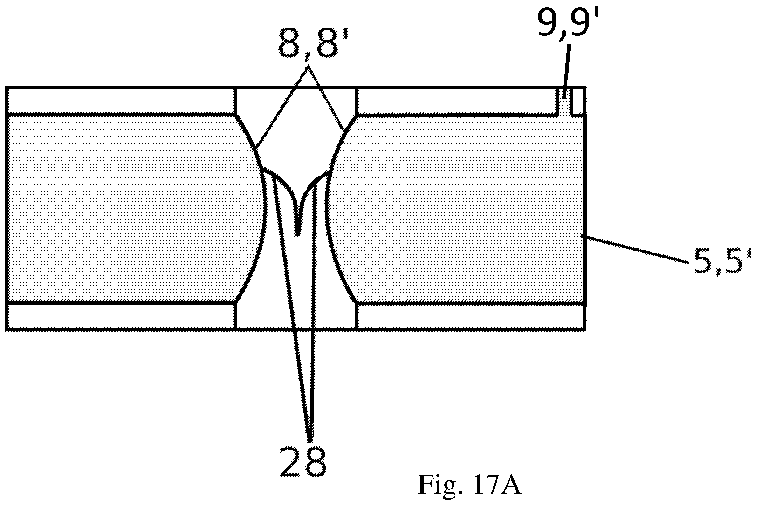

Comme le montre la figure 17A, il peut aussi être prévu que les membranes internes élastiquement déformable 8, 8', 23' des parties ou segments 5, 5', 23 formant mors, définissant un tube 4 de préhension et venant en contact avec l'objet allongé 2, comportent des formations saillantes 28 ou des texturations de surface assurant un maintien centré souple de l'objet allongé 2 à l'état dégonflé et rétracté desdits mors, telles que par exemple des lèvres.

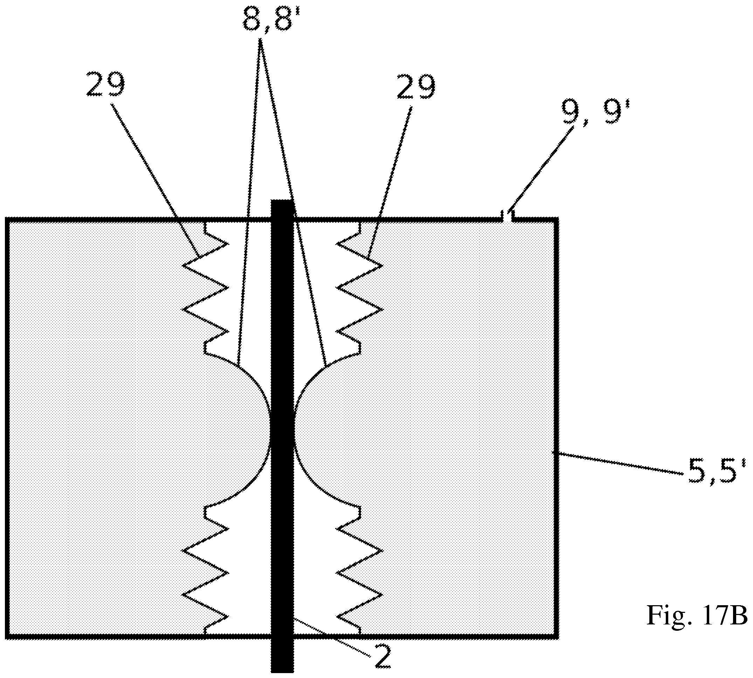

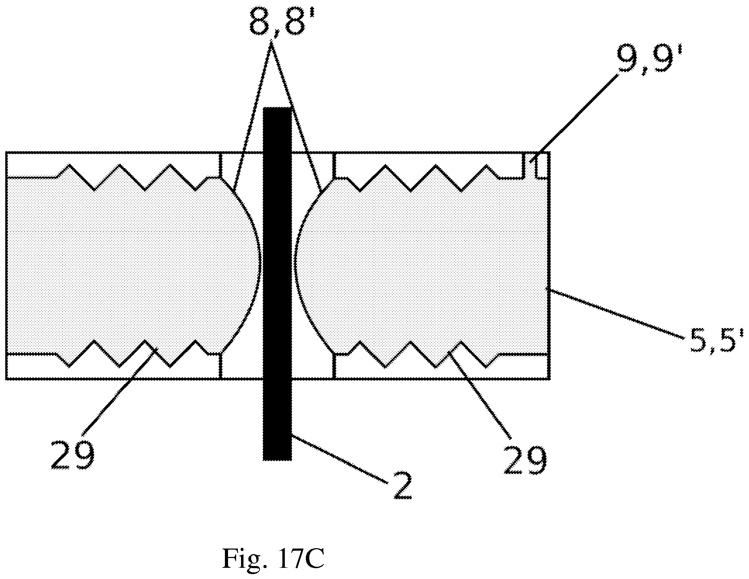

De même, et comme illustrées sur les figures 17B et 17C, l'invention peut également prévoir que la paroi d'au moins un(e) des parties ou segments 5, 5', 23 formant mors, préférentiellement les parois de toutes ces parties, comporte(nt) des portions 29 déformables élastiquement à l'état gonflé, actif ou serré dudit ou desdits mors, fournissant une compliance structurelle radiale et/ou axiale au moins limitée à l'objet allongé 2 saisi par ce(s) mors.

Ces portions 29, par exemple en soufflets, autorisent ainsi une compliance radiale ou axiale augmentée.

Afin de fournir un module opératoire intégré complet, de faible taille, le dispositif 1 peut comprendre une électrovanne 27 de commande de l'injection de fluide, accolée constructivement à l'un(e) 5 des parties ou segments d'extrémité formant mors 5, 5'.

L'homme du métier comprend que le dispositif 1 selon l'invention permet de contrôler le mouvement de translation qui est l'un des mouvements fondamentaux de tout système robotique, et réalise en même temps la motorisation de ce mouvement. L'intégration de deux fonctions en un composant, grâce à la raideur et à la construction du dispositif 1 dans les directions autres que la direction de mouvement permet une compacité unique.

Le dispositif 1 proposé offre en outre intrinsèquement une compliance axiale, liée à la caractéristique de la chambre centrale 6' qui peut être modifiée à la conception. Cette compliance est un élément important pour la sécurité comme exposé plus haut.

Des compliances constructives additionnelles peuvent être intégrées par des configurations particulières des parties 5, 5' et 6 comme indiqué précédemment les modes de réalisation proposés s'appuyant sur des matériaux polymère et sur l'excellente puissance massique des technologies pneumatiques permettent, par exemple, de proposer un composant très léger pour former des bras robotiques de faible masse.

Enfin, le dispositif 1 forme un actionneur qui constitue un moyen sûr et fiable pour contrôler les efforts moteurs en modulant simplement une grandeur qui est la pression à l'intérieur de l'ensemble de la chambre centrale et des mors : ce qui est en fait un limiteur d'effort mécanique particulièrement fiable.

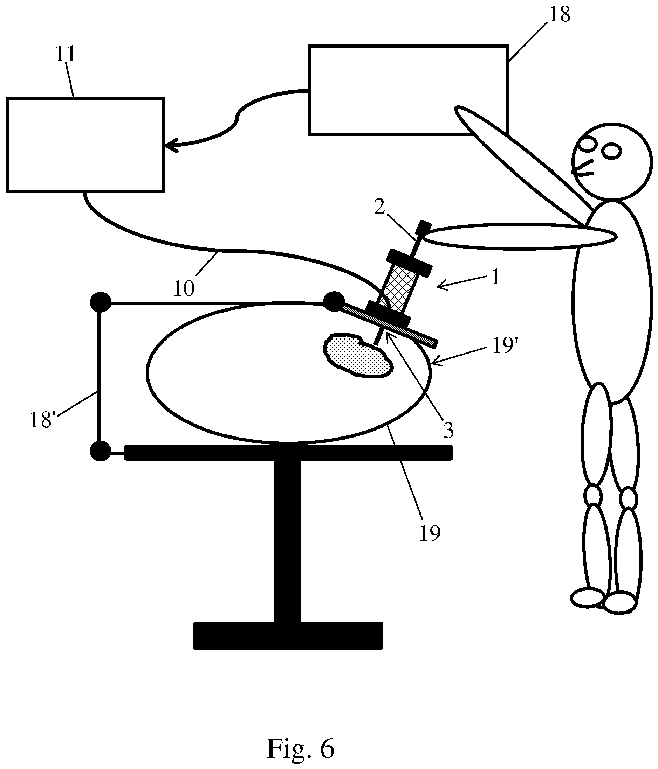

L'invention concerne également, comme le montre schématiquement la figure 6, un système de maintien et de déplacement séquentiel contrôlé d'un objet allongé 2 du type instrument ou outil chirurgical ou médical de forme oblongue, destiné à être introduit dans le corps d'un patient 19.

Ce système comprend, d'une part, au moins un dispositif pneumatique de maintien et de déplacement porté par un support spécifique 18' ou solidarisé au patient 19, et disposé en contact direct avec la peau 19' de ce dernier, d'autre part, au moins une source de fluide gazeux sous pression et, enfin, une interface de commande homme-machine 18, faisant éventuellement partie d'une installation médicale.

Ce système est caractérisé en ce que ledit au moins un dispositif pneumatique de maintien et de déplacement consiste en un dispositif 1 tel que décrit précédemment, son corps creux 1' étant maintenu au niveau de sa partie d'extrémité frontale 5.

L'invention porte également sur un système commandé, préférentiellement automatisé, d'insertion et éventuellement d'assemblage ou de montage, par mouvement translatif, éventuellement combiné à un mouvement rotatif d'un objet allongé 2, du type pièce constructive, outil ou instrument, dans un passage, un orifice ou un logement de réception adapté d'un(e) autre objet, pièce, partie, élément ou analogue (non représenté).

Ce système est caractérisé en qu'il comprend au moins un dispositif 1 pneumatique de maintien et de déplacement guidé tel que décrit ci-dessus ledit dispositif 1 étant monté sur un moyen support, éventuellement mobile, tel qu'un bras robot ou un robot collaboratif, ledit dispositif 1 faisant le cas échéant partie intégrante dudit bras robot ou robot.

En vue d'augmenter la force de poussée applicable à l'objet 2, il peut être prévu que le système comprenne au moins deux dispositifs pneumatiques 1 de maintien et de déplacement, arrangés de manière alignée selon leur direction axiale DA (i.e. les directions axiales DA des dispositifs 1 sont confondues) et traversés chacun par l'objet allongé 2 à maintenir et à déplacer automatiquement sur commande, lesdits dispositifs 1 étant alimentés en fluide sous pression de manière coordonnée (non représenté).

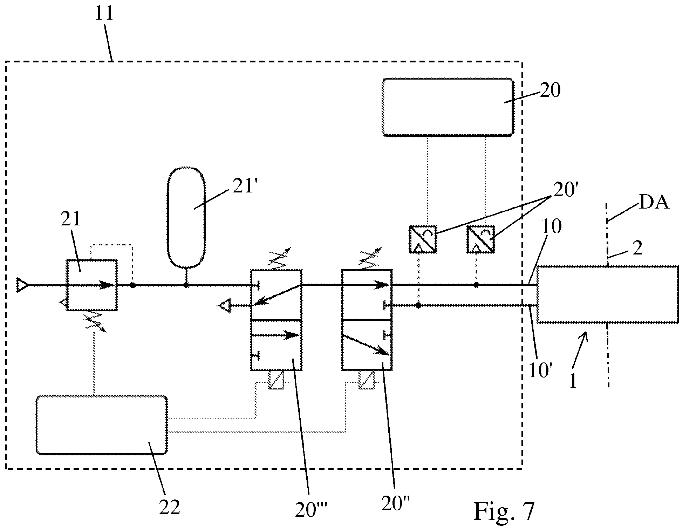

La figure 7 illustre, à titre d'exemple, un système d'alimentation contrôlée formant source de fluide (ici de l'air sous pression) pour le dispositif 1. Le système représenté comprend : une interface de mesure 20 reliée à des capteurs de pression 20' surveillant les lignes 10, 10' ; une vanne 20" de sélection de direction de déplacement ; une vanne 20'" de génération du profil temporel de pression d'alimentation ; un régulateur de pression 21 relié au réseau d'alimentation et associé à un accumulateur 21' ; un dispositif 22 de contrôle et de pilotage du système.

Enfin, l'invention vise également un procédé d'insertion d'un instrument ou outil allongé dans le corps d'un sujet au moyen d'un système mentionné ci-dessus.

Ce procédé est caractérisé en ce qu'il consiste à mettre en place au moins un dispositif pneumatique 1 de maintien et de déplacement en alignant sa direction axiale DA avec un orifice d'entrée ménagé préalablement dans la peau 19' du sujet 19, à mettre ensuite en place l'instrument ou l'outil dans le passage axial 4 du corps creux 1' du dispositif 1 jusqu'à une position d'introduction prédéterminée, à réaliser ensuite un enfoncement progressif et séquentiel dudit outil ou instrument 2 en réalisant, pour chaque pas élémentaire de déplacement, successivement une phase d'injection de fluide sous pression et une phase d'évacuation dudit fluide, la phase d'injection étant interrompue lorsque la chambre d'extrémité arrière 7' du corps creux 1' multichambre a atteint une pression interne correspondant à sa pression opérationnelle, et à répéter le cycle des deux phases précitées autant de fois que nécessaire pour atteindre l'amplitude de déplacement en translation, voire le degré d'enfoncement, souhaité.

Enfin, l'invention porte aussi sur un procédé d'insertion d'un objet allongé 2, du type pièce constructive, outil ou instrument, dans un orifice ou un passage d'une pièce au moyen du système commandé précité.

Ce procédé est caractérisé en ce qu'il consiste à mettre en place au moins un dispositif pneumatique 1 de maintien et de déplacement en alignant sa direction axiale DA avec l'ouverture d'entrée de l'orifice ou du passage concerné, l'objet allongé 2 étant en place dans le passage axial 4 du corps creux 1' du dispositif 1, à réaliser ensuite un enfoncement progressif et séquentiel dudit objet 2 en réalisant, pour chaque pas élémentaire de déplacement, successivement une phase d'injection de fluide sous pression et une phase d'évacuation dudit fluide, la phase d'injection étant interrompue lorsque la chambre d'extrémité arrière 5' du corps creux 1' multichambre a atteint une pression interne correspondant à sa pression opérationnelle, et à répéter le cycle des deux phases précitées autant de fois que nécessaire pour atteindre l'amplitude de déplacement en translation, voire le degré d'enfoncement, souhaité.

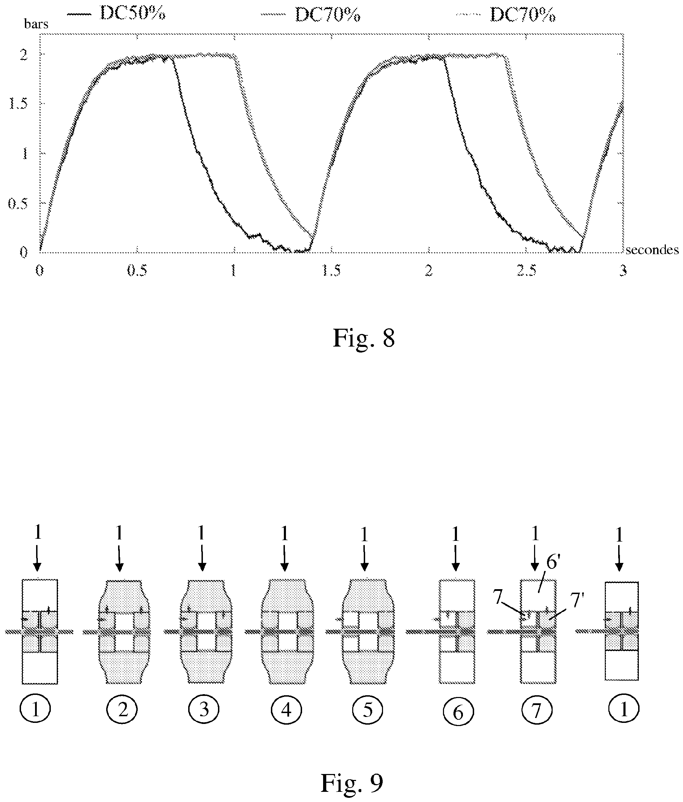

Lorsqu'une prise continue du dispositif sur l'objet 2 tout au long du mouvement d'enfoncement est recherchée, il peut être prévu que la phase d'injection du cycle suivant débute alors que la phase d'évacuation du cycle en cours n'est pas achevée (voir figures 8 et 9).

La différence entre la cinématique de la figure 1 (avec relâchement de l'objet 2) et celle de la figure 9 (sans relâchement) ressort d'une comparaison des représentations des étapes 1 de la figure 1 (phases de relâchement) avec les étapes 1 et 7 de la figure 9. Sur la figure 9, on note que lorsque la chambre 7' commence à se vider (serrage encore effectif), la chambre 7 est déjà en train de se gonfler (étape 7). Le serrage au niveau du mors 5 va alors se raffermir tandis que le serrage au niveau du mors 5' continue à se relâcher pour autoriser le déploiement par allongement axial de la chambre 6' et le glissement du mors 5' sur l'objet 2 (étape 2).

Bien entendu, l'invention n'est pas limitée aux modes de réalisation décrits et représentés aux dessins annexés. Des modifications restent possibles, notamment du point de vue de la constitution des divers éléments ou par substitution d'équivalents techniques, sans sortir pour autant du domaine de protection de l'invention défini par les revendications.

## Revendications

1. Dispositif pneumatique de maintien et de déplacement d'un objet allongé, ledit dispositif comprenant un corps creux de forme générale annulaire ou tubulaire définissant une direction axiale, traversé par l'objet allongé au niveau d'un passage axial et constitué par deux parties ou segments d'extrémité arrangé(e)s de manière alignée dans la direction axiale et formant mors et un(e) partie ou segment médian(e) ou central(e) reliant les deux parties ou segments formant mors entre eux, avec faculté de déplacement relatif de l'un par rapport à l'autre dans la direction axiale sous l'effet d'une déformation contrôlée d'au moins une portion de ladite partie ou dudit segment médian(e) ou central(e),
dispositif (1) **caractérisé en ce que** tou(te)s les différent(e)s parties ou segments (5, 5', 6) du corps creux (1') définissent des chambres (7, 7', 6') respectives,
**en ce que** les partie(s) ou segment(s) d'extrémité (5, 5') formant mors comportent, en tant que portions de parois (8, 8') destinées à venir en contact avec l'objet allongé (2) en phase de serrage, des membranes élastiquement déformables,
et **en ce que** les chambres (7, 7') des deux parties ou segments d'extrémité (5, 5') sont en communication fluidique avec la chambre (6') de la partie ou du segment médian(e) ou central(e) (6) par l'intermédiaire chacun(e) d'au moins un moyen calibré d'écoulement (9, 9') respectif, l'injection de fluide gazeux sous pression s'effectuant par le biais d'une unique ligne d'alimentation (10, 10') reliée à une source contrôlée de fluide sous pression (11) et débouchant dans la chambre (7, 7') de l'un(e) (5 ou 5') des deux parties ou segments d'extrémité (5, 5'), ce en fonction du sens de déplacement en translation souhaité pour l'objet allongé (2) présent dans le corps creux (1').

2. Dispositif selon la revendication 1, **caractérisé en ce que** les parties ou segments d'extrémité (5, 5') formant mors, respectivement frontal et arrière en fonction du sens de déplacement positif de l'objet allongé (2), délimitent des chambres (7, 7') de forme annulaire ou torique de révolution autour de la direction axiale (AD), et présentant une structure sensiblement indéformable, axialement et radialement, dans la gamme de pressions opérationnelles de fluide injecté en fonctionnement normal du dispositif (1), à l'exception de membranes élastiquement déformables formant des manchons (8, 8') internes constituant les logements traversants des mors pour l'objet allongé (2), formant par alignement le passage axial (4) du corps creux (1') multichambre.

3. Dispositif selon l'une quelconque des revendications 1 et 2, **caractérisé en ce que** la partie ou le segment médian(e) ou central(e) (6) présente au moins une portion de paroi commune (12, 12') avec respectivement chacun(e) des deux parties ou segments d'extrémité (5, 5'), chacune desdites portions de parois communes (12, 12') participant ainsi simultanément à la délimitation partielle de l'une des chambres d'extrémité (7, 7') et à celle de la chambre médiane ou centrale (6'), et **en ce que** les moyens calibrés d'écoulement (9, 9') consistent en des trous traversants ménagés dans lesdites portions de parois communes (12, 12') et présentant des formes et des tailles déterminées, fonction du séquencement souhaité pour le gonflement et le dégonflage des chambres (7, 6', 7') déterminant l'amplitude et/ou la vitesse du mouvement de déplacement en translation de l'objet allongé (2) considéré.

4. Dispositif selon l'une quelconque des revendications 1 à 3, **caractérisé en ce que** le calibrage de chacun des moyens d'écoulement respectifs (9, 9') entre chacune des chambres d'extrémité (5, 5') et la chambre médiane ou centrale (6), éventuellement différent pour chaque moyen (9, 9'), est réglé de telle manière que, par contrôle passif de la circulation du fluide gazeux injecté par l'unique ligne d'alimentation (10, 10'), les deux chambres d'extrémité (5 et 5') peuvent être sous une pression opérationnelle entraînant un double serrage effectif de l'objet (2) à déplacer, lorsque la chambre médiane ou centrale (6) est sous pression opérationnelle et dans son état déformé.

5. Dispositif selon l'une quelconque des revendications 1 à 4, **caractérisé en ce que** la partie ou le segment médian(e) ou central(e) (6) du corps creux (1') est au moins partiellement formé(e) ou recouvert(e) d'une couche (13) d'un matériau auxétique, c'est-à-dire avec un coefficient de Poisson négatif.

6. Dispositif selon l'une quelconque des revendications 1 à 5, **caractérisé en ce que** le corps creux (1') multichambre avec ses trois sorties ou segments (5, 5') est entièrement réalisé en matériau(x) polymère(s), préférentiellement en tant que pièce monobloc ou réalisée d'un seul tenant, par exemple par impression tridimensionnelle, les membranes déformables (8, 8') des mors (5, 5') étant éventuellement rapportées par surmoulage d'un matériau polymère distinct spécifique.

7. Dispositif selon l'une quelconque des revendications 1 à 6, **caractérisé en ce qu'**un conduit (10, 10') apte et destiné à former sélectivement une ligne d'alimentation ou une ligne d'évacuation est relié à chacune des chambres (7, 7') des parties ou segments d'extrémité (5, 5').

8. Dispositif selon l'une quelconque des revendications 1 à 7, **caractérisé**
**en ce que** la partie ou le segment médian(e) ou central(e) (6) définit une chambre (6') annulaire ou torique et s'étend périphériquement, et radialement extérieurement, autour des deux parties ou segments d'extrémité (5, 5') pour aboutir à un corps creux (1') multichambre de forme discoïdale et **en ce qu'**elle relie ces parties ou segments (5, 5') entre eux/elles par une liaison élastiquement déformable dans la direction axiale (DA), les deux parties ou segments d'extrémité (5, 5') étant en contact mutuel dans ladite direction axiale (DA) à l'état dégonflé de la partie ou du segment médian(e) ou central(e) (6) et les deux parties ou segments d'extrémité (5, 5') étant situé(e)s à distance l'un(e) de l'autre à l'état gonflé de ladite partie ou dudit segment médian(e) ou central(e) (6), sous une pression suffisante pour entraîner une déformation de ce(tte) dernier(e),
**en ce que** la partie ou le segment médian(e) ou central(e) (6) du corps creux (1') multichambre est constitué(e), d'une part, par une portion de paroi tubulaire interne (14) pouvant être déformée élastiquement dans la direction axiale (DA) et reliant les deux parties ou segments d'extrémité (5, 5') entre eux(elles) et, d'autre part, par une portion de paroi à section en U externe (15), de révolution autour de la direction axiale (DA) et formant par coopération complémentaire avec la portion de paroi tubulaire interne (14), la chambre médiane ou centrale (6') annulaire ou torique du corps creux (1'), les deux ailes (15') du U étant également reliées par leurs extrémités libres aux deux parties ou segments d'extrémité (5, 5') et étant déformables élastiquement par flexion vers l'extérieur du U dans la direction axiale (DA), sous l'effet d'une surpression suffisante dans la chambre centrale ou médiane (6'), et
**en ce que** les zones (15") de la portion de paroi à section en U externe (15) qui correspondent aux ailes (15') dudit U sont formées ou recouverts, préférentiellement sur leur face interne, par une couche (13) de matériau auxétique.

9. Dispositif selon l'une quelconque des revendications 1 à 7, **caractérisé en ce que** la partie ou le segment médian(e) ou central(e) (6) comprend une chambre tubulaire cylindrique (6') s'étendant dans la direction axiale (DA) et de révolution autour de cette dernière, ladite chambre (6') comportant une paroi extérieure (16) déformable élastiquement et étant en communication fluidique calibrée avec les chambres (7, 7') des parties ou segments d'extrémité (5, 5'), **en ce que** ladite paroi (16) est recouverte extérieurement d'une structure cylindrique à propriété auxétique (16'), qui est formée d'un réseau maillé à motif répétitif de formations élémentaires (17) et qui prend appui sur et est solidaire de deux butées discoïdales (17') opposées, également reliées à la paroi déformable (16) précitée et formant des portions de paroi communes (12, 12') avec ou reliées aux parties ou segments d'extrémité (5, 5'), les composantes constitutives précitées de la partie ou du segment médian(e) (6) étant mutuellement arrangés de telle manière que la mise sous pression opérationnelle de la chambre médiane ou centrale (6') entraîne par action de la paroi (16) sur le réseau (16') un écartement mutuel des butées discoïdales (17') dans la direction axiale (DA), et donc des parties ou segments d'extrémité (5, 5') respectivement solidaires desdites butées (17'), les formations élémentaires (17) constituant avantageusement ensemble un réseau (16') à motif en nids d'abeilles inversés.

10. Dispositif selon l'une quelconque des revendications 1 à 9, **caractérisée en ce que** les parties ou segments d'extrémité (5, 5') et la partie ou le segment médian(e) ou central(e) (6) sont mutuellement alignées entre elles (eux) et toutes traversé(e)s par l'objet allongé (2), la direction axiale (DA) du corps creux (1') et l'axe de translation de l'objet allongé (2) étant confondus.

11. Dispositif selon l'une quelconque des revendications 1 à 10, **caractérisé en ce que** les parties ou segments d'extrémité (5, 5') sont mutuellement alignées entre elles (eux) et traversé(e)s tou(te)s deux par l'objet allongé (2), l'axe de translation de l'objet allongé (2) étant confondu avec l'axe d'alignement desdit(e)s parties ou segments d'extrémités (5, 5') correspondant à la direction axiale (DA), la partie ou le segment médian(e) ou central(e) (6) étant décalé(e) latéralement ou radialement par rapport à cette direction (DA) et n'étant pas traversé par l'objet allongé (2).

12. Dispositif selon l'une quelconque des revendication 1 à 7, **caractérisé en ce que** la partie ou le segment médian(e) ou central(e) (6) comprend trois chambres ou parties fonctionnelles contiguës (22, 22', 23), à savoir, d'une part, deux chambres (22, 22') aptes et destinées à assurer une fonction de déplacement sous l'effet d'une déformation d'au moins une portion de leur paroi résultant d'une modification de leur pression interne, et accolées chacune respectivement à l'un(e) des parties ou segments d'extrémité (5, 5') formant mors gonflables, avec lesquel(le)s ils (elles) sont respectivement en communication fluidique par un moyen calibré d'écoulement (9, 9'), et, d'autre part, une partie ou chambre fonctionnelle médiane (23) formant mors, située entre les deux chambres ou parties fonctionnelles (22 et 22') précitées et en communication fluidique par un moyen calibré d'écoulement respectif (24, 24') avec chacune d'entre elles, l'ensemble des cinq parties (5, 5', 22, 22', 23) formant une structure cylindrique, une première des deux chambres fonctionnelles (22) étant avantageusement configurée pour assurer un déplacement en rotation autour de la direction axiale (AD) de l'élément allongé (2) serré par un (5) des deux mors d'extrémité (5, 5') et la seconde des deux chambres fonctionnelles (22') étant avantageusement configurée pour assurer un déplacement en translation le long de la direction axiale (AD) de l'objet allonge" (2) serré par le mors médian (23) et l'autre mors d'extrémité (5').

13. Dispositif selon l'une quelconque des revendications 1 à 12, **caractérisé en ce qu'**il comprend au moins un moyen de réglage (25) d'au moins un moyen calibré d'écoulement (9, 9', 24, 24'), en particulier de sa section de passage, un tel moyen de réglage (25) étant préférentiellement associé à chaque moyen d'écoulement (9, 9', 24, 24'), le ou chaque moyen de réglage (25), à actionnement manuel ou motorisé, comprenant avantageusement un élément mobile d'obturation variable de l'orifice calibrée (9, 9', 24, 24') formant le moyen d'écoulement considéré.

14. Dispositif selon l'une quelconque des revendications 1 à 13, **caractérisé en ce qu'**il comprend un moyen capteur (26), rapporté ou intégré à la partie ou au segment médian(e) ou central(e) (6) et apte et destiné à délivrer un signal fonction de l'élongation de cette partie ou de ce segment dans la direction axiale (AD) et/ou autour de cette dernière.

15. Dispositif selon l'une quelconque des revendications 1 à 14, **caractérisé en ce que** les membranes internes élastiquement déformable (8, 8', 23') des parties ou segments (5, 5', 23) formant mors, définissant un tube et venant en contact avec l'objet allongé (2), comportent des formations saillantes (28) ou des texturations de surface assurant un maintien centré souple de l'objet allongé (2) à l'état dégonflé et rétracté desdits mors, telles que par exemple des lèvres, la paroi d'au moins un(e) des parties ou segments (5, 5', 23) formant mors, préférentiellement les parois de toutes ces parties, comportant avantageusement des portions (29) déformables élastiquement à l'état gonflé, actif ou serré dudit ou desdits mors, fournissant une compliance structurelle radiale et/ou axiale au moins limitée à l'objet allongé (2) saisi par ce(s) mors.

16. Dispositif selon l'une quelconque des revendications 1 à 15, **caractérisé en ce qu'**il comprend une électrovanne (27) de commande de l'injection de fluide, accolée constructivement à l'un(e) (5) des parties ou segments d'extrémité formant mors (5, 5').

17. Système de maintien et de déplacement séquentiel contrôlé d'un objet allongé du type instrument ou outil chirurgical ou médical de forme oblongue, destiné à être introduit dans le corps d'un patient,
ledit système comprenant, d'une part, au moins un dispositif pneumatique de maintien et de déplacement porté par un support spécifique ou solidarisé au patient, et disposé en contact direct avec la peau de ce dernier, d'autre part, au moins une source de fluide gazeux sous pression et, enfin, une interface de commande homme-machine, faisant éventuellement partie d'une installation médicale,
système **caractérisé en ce que** ledit au moins un dispositif pneumatique de maintien et de déplacement consiste en un dispositif (1) selon l'une quelconque des revendications 1 à 16, son corps creux (1') étant maintenu au niveau de sa partie d'extrémité frontale (5).

18. Système commandé, préférentiellement automatisé, d'insertion et éventuellement d'assemblage ou de montage, par mouvement translatif, éventuellement combiné à un mouvement rotatif d'un objet allongé, du type pièce constructive, outil ou instrument, dans un passage, un orifice ou un logement de réception adapté d'un(e) autre objet, pièce, partie, élément ou analogue,
système caractérisé en qu'il comprend au moins un dispositif (1) pneumatique de maintien et de déplacement guidé selon l'une quelconque des revendications 1 à 16, ledit dispositif (1) étant monté sur un moyen support, éventuellement mobile, tel qu'un bras robot ou un robot collaboratif, ledit dispositif (1) faisant le cas échéant partie intégrante dudit bras robot ou robot.

## Patentansprüche

1. Pneumatische Vorrichtung zum Halten und Bewegen eines länglichen Gegenstandes, wobei die genannte Vorrichtung einen im Wesentlichen ring- oder rohrförmigen Hohlkörper mit einer Längsachse umfasst, der vom länglichen Gegenstand im Bereich eines axialen Durchganges durchquert wird und aus zwei Endbereichen oder -abschnitten besteht, die in der Achsenrichtung zueinander ausgerichtet angeordnet sind und Spannbacken darstellen, und einem Mittelbereich oder - abschnitt, der die beiden Spannbacken darstellenden Bereiche oder Abschnitte miteinander verbindet, mit der Möglichkeit, sich unter der Einwirkung einer geregelten Verformung mindestens eines Teils des genannten Mittelbereichs oder -abschnitts relativ zueinander in der Achsenrichtung zu bewegen,
Vorrichtung (1), **dadurch gekennzeichnet, dass** alle Bereiche oder Abschnitte (5, 5', 6) des Hohlkörpers (1') zugehörige Kammern (7, 7', 6') definieren,
dadurch, dass die Spannbacken darstellenden Endbereiche oder -abschnitte (5, 5') als Wandteile (8, 8'), die dazu bestimmt sind, mit dem länglichen Gegenstand (2) in der Einspannphase in Berührung zu kommen, elastisch verformbare Membranen aufweisen,
dadurch, dass die Kammern (7, 7') der beiden Endbereiche oder -abschnitte (5, 5') jeweils durch mindestens ein zugehöriges kalibriertes Abflussmittel (9, 9') mit der Kammer (6') des Mittelbereichs oder -abschnitts (6) kommunizieren, wobei die Einleitung gasförmigen Druckfluides mit Hilfe einer einzigen Speiseleitung (10, 10') erfolgt, die an eine geregelte Druckfluidquelle (11) angeschlossen ist und in die Kammer (7, 7') eines (5 oder 5') der beiden Endbereiche oder -abschnitte (5, 5') mündet, dies in Abhängigkeit von der gewünschten Bewegungsrichtung des länglichen Gegenstandes (2), der sich im Hohlkörper (1') befindet.

2. Vorrichtung nach Patentanspruch 1, **dadurch gekennzeichnet, dass** die, je nach der positiven Bewegungsrichtung des länglichen Gegenstandes (2) vordere oder hintere, Spannbacken darstellenden Endbereiche oder -abschnitte (5, 5') um die Längsachse (AD) rotationssymmetrische, ringförmige oder torische Kammern (7, 7') umgrenzen und einen im Bereich der Arbeitsdrücke des eingeleiteten Fluides bei Normalbetrieb der Vorrichtung (1) im Wesentlichen axial oder radial nicht verformbaren Aufbau aufweisen, mit Ausnahme elastisch verformbarer Membranen, die Innenhülsen (8, 8') bilden, die die durchgehenden Aufnahmen der Spannbacken für den länglichen Gegenstand (2) bilden, die durch Ausrichtung den axialen Durchgang (4) des Mehrkammerhohlkörpers (1') bilden.

3. Vorrichtung nach irgendeinem der Patentansprüche 1 und 2, **dadurch gekennzeichnet, dass** der Mittelbereich oder -abschnitt (6) jeweils mindestens einen gemeinsamen Wandteil (12, 12') mit jedem der beiden Spannbacken darstellenden Endbereiche oder -abschnitte (5, 5') aufweist, wobei jeder der genannten gemeinsamen Wandteile (12, 12') auf diese Weise gleichzeitig an der partiellen Begrenzung einer der Endkammern (7, 7') und an der der Mittelkammer (6') teilhat, und dadurch, dass die kalibrierten Abflussmittel (9, 9') in durchgehenden Löchern bestehen, die in den genannten gemeinsamen Wandteilen (12, 12') ausgebildet sind und festgelegte Formen und Größen aufweisen, die Funktion der gewünschten Abfolge von Füllung und Leerung der Kammern (7, 6', 7') sind, die die Amplitude und/oder die Geschwindigkeit der Verschiebungsbewegungen des betrachteten länglichen Gegenstandes (2) bestimmen.

4. Vorrichtung nach irgendeinem der Patentansprüche 1 bis 3, **dadurch gekennzeichnet, dass** die Kalibrierung jedes der entsprechenden Abflussmittel (9, 9') zwischen jeder der Endkammern (5, 5') und der Mittelkammer (6), die gegebenenfalls bei jedem Mittel (9, 9') verschieden sind, derart geregelt ist, dass durch passive Regulierung der Strömung des durch die einzige Speiseleitung (10, 10') eingeleiteten gasförmigen Fluides beide Endkammern (5 und 5') unter einem Arbeitsdruck stehen können, der eine effektive doppelte Einspannung des zu bewegenden Gegenstandes (2) bewirkt, wenn die Mittelkammer (6) unter Arbeitsdruck steht und in ihrem verformten Zustand ist.

5. Vorrichtung nach irgendeinem der Patentansprüche 1 bis 4, **dadurch gekennzeichnet, dass** der Mittelbereich oder -abschnitt (6) des Hohlkörpers (1') mindestens teilweise aus einer Schicht (13) eines auxetischen Materials, d.h. mit negativer Poissonzahl, besteht oder damit beschichtet ist.

6. Vorrichtung nach irgendeinem der Patentansprüche 1 bis 5, **dadurch gekennzeichnet, dass** der Mehrkammerhohlkörper (1') mit seinen drei Bereichen oder Abschnitten (5, 5') vollständig aus Polymerwerkstoff(en) gefertigt ist, vorzugsweise einstückig, beispielsweise durch dreidimensionales Drucken, wobei die verformbaren Membranen (8, 8') der Spannbacken (5, 5') gegebenenfalls durch Aufformen eines anderen, spezifischen Polymerwerkstoffes angefügt sind.

7. Vorrichtung nach irgendeinem der Patentansprüche 1 bis 6, **dadurch gekennzeichnet, dass** eine Leitung (10, 10'), geeignet und dazu bestimmt, wahlweise eine Speiseleitung oder eine Abflussleitung zu bilden, an jede der Kammern (7, 7') der Endbereiche oder -abschnitte (5, 5') angeschlossen ist.

8. Vorrichtung nach irgendeinem der Patentansprüche 1 bis 7, **dadurch gekennzeichnet, dass**
der Mittelbereich oder -abschnitt (6) eine ringförmige oder torische Kammer (6') definiert und sich peripher und radial außen um die beiden Endbereiche oder -abschnitte (5, 5') erstreckt, um einen scheibenförmigen Mehrkammerhohlkörper (1') zu erzielen, und dadurch, dass er diese Bereiche oder Abschnitte (5, 5') durch eine elastisch verformbare Verbindung in der Achsenrichtung (DA) miteinander verbindet, wobei die beiden Endbereiche oder -abschnitte (5, 5') im unaufgeblasenen Zustand des Mittelbereichs oder -abschnitts (6) in der genannten Achsenrichtung (DA) miteinander in Berührung stehen und die beiden Endbereiche oder -abschnitte (5, 5') im aufgeblasenen Zustand des genannten Mittelbereichs oder -abschnitts (6) unter einem ausreichenden Druck, um dessen Verformung zu bewirken, sich in einem Abstand voneinander befinden,
dadurch, dass der Mittelbereich oder -abschnitt (6) des Mehrkammerhohlkörpers (1') einerseits aus einem inneren, rohrförmigen Wandteil (14) besteht, der in der Achsenrichtung (DA) elastisch verformbar ist und die beiden Endbereiche oder -abschnitte (5, 5') miteinander verbindet, und andererseits aus einem äußeren, um die Achsenrichtung (DA) rotationssymmetrischen Wandteil (15) mit U-förmigem Querschnitt, der in komplementärer Zusammenwirkung mit dem inneren, rohrförmigen Wandteil (14) die ringförmige oder torische Mittelkammer (6') des Mehrkammerhohlkörpers (1') bildet, wobei die beiden Arme (15') des U an ihren freien Enden ebenfalls mit den beiden Endbereichen oder -abschnitten (5, 5') verbunden sind und unter der Wirkung eines ausreichenden Überdruckes in der Mittelkammer (6') durch Biegen nach außerhalb des U in der Achsenrichtung (DA) elastisch verformbar sind, und
dadurch, dass die Zonen (15") des äußeren Wandteils (15) mit U-förmigem Querschnitt, die den Armen (15') des genannten U entsprechen, aus auxetischem Werkstoff geformt oder mit einer Schicht (13) davon beschichtet sind, vorzugsweise auf ihrer Innenseite.

9. Vorrichtung nach irgendeinem der Patentansprüche 1 bis 7, **dadurch gekennzeichnet, dass** der Mittelbereich oder -abschnitt (6) eine rohrförmige zylindrische Kammer (6') umfasst, die sich in der Achsenrichtung (DA) erstreckt und um diese rotationssymmetrisch ist, wobei die genannte Kammer (6') eine elastisch verformbare Außenwand (16) aufweist, die mit den Kammern (7, 7') der Endbereiche oder -abschnitte (5, 5') kalibriert kommuniziert, dadurch, dass die genannte Wand (16) außen mit einer zylindrischen Struktur mit auxetischen Eigenschaften (16') beschichtet ist, die aus einem Maschengitter aus repetitiven Elementarbildungen (17) besteht und die sich an zwei einander gegenüberliegenden scheibenförmigen Anschlägen (17') abstützt und mit diesen fest verbunden ist, die ebenfalls mit der genannten verformbaren Wand (16) verbunden sind und gemeinsame Wandteile (12, 12') mit den Endbereichen oder -abschnitten (5, 5') bilden oder mit diesen verbunden sind, wobei die oben genannten Bestandteile des Mittelbereichs oder - abschnitts (6) derart zueinander angeordnet sind, dass das Setzen der Mittelkammer (6') unter Arbeitsdruck durch Einwirkung der Wand (16) auf das Gitter (16') zu einer Entfernung der scheibenförmigen Anschläge (17') voneinander in der Achsenrichtung (DA) führt und also der Endbereiche oder -abschnitte (5, 5'), die je mit den genannten Anschlägen (17') fest verbunden sind, wobei die Elementarbildungen (17) vorteilhafterweise zusammen ein Gitter (16') mit invertiertem Bienenwabenmuster bilden.

10. Vorrichtung nach irgendeinem der Patentansprüche 1 bis 9, **dadurch gekennzeichnet, dass** die Endbereiche oder -abschnitte (5, 5') und der Mittelbereich oder -abschnitt (6) zueinander ausgerichtet sind und alle vom länglichen Gegenstand (2) durchquert werden, wobei die Achsenrichtung (DA) des Hohlkörpers (1') und die Verschiebungsachse des länglichen Gegenstandes (2) zusammenfallen.

11. Vorrichtung nach irgendeinem der Patentansprüche 1 bis 10, **dadurch gekennzeichnet, dass** die Endbereiche oder -abschnitte (5, 5') zueinander ausgerichtet sind und beide vom länglichen Gegenstand (2) durchquert werden, wobei die Verschiebungsachse des länglichen Gegenstandes (2) mit der Ausrichtungsachse der genannten Endbereiche oder -abschnitte (5, 5') zusammenfällt, die der Achsenrichtung (DA) entspricht, wobei der Mittelbereich oder -abschnitt (6) seitlich oder radial gegenüber dieser Richtung (DA) versetzt ist und nicht vom länglichen Gegenstand (2) durchquert wird.

12. Vorrichtung nach irgendeinem der Patentansprüche 1 bis 7, **dadurch gekennzeichnet, dass** der Mittelbereich oder -abschnitt (6) drei aneinander anschließende Kammern oder Funktionsteile (22, 22', 23) aufweist, und zwar einerseits zwei Kammern (22, 22'), die geeignet und dazu bestimmt sind, unter der Wirkung einer Verformung mindestens eines Teils ihrer Wand infolge einer Änderung ihres Innendruckes eine Verschiebungsfunktion sicherzustellen, und die jeweils an einen der Endbereiche oder -abschnitte (5, 5') angefügt sind, die aufblasbare Spannbacken bilden, mit denen sie durch ein jeweils zugehöriges kalibriertes Abflussmittel (9, 9') kommunizieren, und andererseits einen Mittelteil oder -funktionskammer (23), der eine Spannbacke bildet, der zwischen den beiden genannten Kammern oder Funktionsteilen (22 und 22') angeordnet ist und durch ein jeweils zugehöriges kalibriertes Abflussmittel (24, 24') mit jeder von ihnen kommuniziert, wobei die Gesamtheit der fünf Teile (5, 5', 22, 22', 23) ein zylindrisches Gebilde bildet, wobei eine erste der beiden Funktionskammern (22) vorteilhafterweise dafür ausgebildet ist, eine Rotationsbewegung um die Achsenrichtung (AD) des länglichen Gegenstandes (2) sicherzustellen, der von einer (5) der beiden Endspannbacken (5, 5') eingeklemmt wird, und die andere der beiden Funktionskammern (22') vorteilhafterweise dafür ausgebildet ist, eine Verschiebungsbewegung des länglichen Gegenstandes (2) längs der Achsenrichtung (AD) sicherzustellen, der von der mittleren Spannbacke (23) und der anderen Endspannbacke (5') eingeklemmt wird.

13. Vorrichtung nach irgendeinem der Patentansprüche 1 bis 12, **dadurch gekennzeichnet, dass** sie mindestens ein Reguliermittel (25) mindestens eines kalibrierten Durchflussmittels (9, 9', 24, 24'), insbesondere seines Durchgangsquerschnittes, umfasst, wobei ein derartiges Reguliermittel (25) vorzugsweise jedem Durchflussmittel (9, 9', 24, 24') zugeordnet ist, wobei das oder jedes Reguliermittel (25), mit manueller oder motorisierter Betätigung, vorteilhafterweise ein bewegliches Teil zum veränderbaren Verschluss der kalibrierten Öffnung (9, 9', 24, 24') umfasst, die das betrachtete Durchflussmittel bildet.

14. Vorrichtung nach irgendeinem der Patentansprüche 1 bis 13, **dadurch gekennzeichnet, dass** sie ein Sensormittel (26) umfasst, das am Mittelbereich oder -abschnitt (6) angebracht oder in ihn eingebaut und dafür geeignet und bestimmt ist, ein Signal in Abhängigkeit von der Verlängerung dieses Bereichs oder -abschnitts in der Achsenrichtung (AD) und/oder um diese auszugeben.

15. Vorrichtung nach irgendeinem der Patentansprüche 1 bis 14, **dadurch gekennzeichnet, dass** die elastisch verformbaren inneren Membranen (8, 8', 23') der Spannbacken bildenden Endbereiche oder -abschnitte (5, 5', 23) ein Rohr definieren und mit dem länglichen Gegenstand (2) in Berührung stehen, vorspringende Bildungen (28) oder Oberflächenstrukturen aufweisen, die ein zentriertes, weiches Halten des länglichen Gegenstandes (2) im entleerten und zurückgezogenen Zustand der genannten Spannbacken sicherstellen, wie etwa Lippen, wobei die Wand mindestens eines der Spannbacken bildenden Bereiche oder -abschnitte (5, 5', 23), vorzugsweise die Wände aller dieser Teile, vorteilhafterweise im aufgeblasenen, aktiven oder gespannten Zustand der genannten Spannbacke(n) elastisch verformbare Teile (29) aufweisen, die dem von dieser/n Spannbacke(n) gehaltenen länglichen Gegenstand eine mindestens begrenzte radiale und/oder axiale konstruktive Federung verleihen.

16. Vorrichtung nach irgendeinem der Patentansprüche 1 bis 15, **dadurch gekennzeichnet, dass** sie ein Magnetventil (27) zur Steuerung der Fluideinleitung umfasst, das konstruktiv einem der Spannbacken bildenden Endbereiche oder -abschnitte (5, 5') angefügt ist.

17. System zum Halten und gesteuerten, aufeinanderfolgenden Bewegen eines länglichen Gegenstandes der Art eines chirurgischen oder medizinischen Instrumentes oder Werkzeugs länglicher Form, das dazu bestimmt ist, in den Körper eines Patienten eingeführt zu werden,
wobei das genannte System einerseits mindestens eine pneumatische Vorrichtung zum Halten und Bewegen umfasst, die von einem spezifischen Träger getragen oder am Patienten befestigt wird und in unmittelbarer Berührung mit der Haut des letzteren steht, andererseits mindestens eine Quelle gasförmigen Druckfluides, und schließlich eine Mensch-Maschine-Steuerschnittstelle, die gegebenenfalls Teil einer medizinischen Anlage ist,
System, **dadurch gekennzeichnet, dass** die genannte mindestens eine pneumatische Vorrichtung zum Halten und Bewegen in einer Vorrichtung (1) nach irgendeinem der Patentansprüche 1 bis 16 besteht, wobei deren Hohlkörper (1') im Bereich ihres vorderen Endbereiches (5) gehalten wird.

18. Gesteuertes, vorzugsweise automatisiertes, System zum Einschieben und gegebenenfalls Zusammensetzen oder Anbringen durch Verschiebungsbewegung, gegebenenfalls in Kombination mit einer Rotationsbewegung, eines länglichen Gegenstandes der Art eines Bauteils, Werkzeugs oder Instrumentes, in einen geeigneten Durchgang, Öffnung oder Aufnahme eines anderen Gegenstandes, Bestandteils, Bereiches, Elementes oder dergleichen,
System **dadurch gekennzeichnet, dass** es mindestens eine pneumatische Vorrichtung (1) zum Halten und geführten Bewegen nach irgendeinem der Patentansprüche 1 bis 16 umfasst, wobei die genannte Vorrichtung (1) auf einem gegebenenfalls bewegbaren Tragmittel angebracht ist, wie etwa einem Roboterarm oder einem kollaborativen Roboter, wobei die genannte Vorrichtung (1) gegebenenfalls in den genannten Roboterarm oder Roboter eingebaut ist.

## Claims

1. Pneumatic device for holding and moving an elongate object, said device comprising a hollow body which is of an annular or tubular general shape defining an axial direction, is traversed by the elongate object at an axial passage and is composed of two end parts or segments that are arranged in alignment in the axial direction and form jaws, and a median or central part or segment that connects the two jaw-forming parts or segments to each other, with relative ease of movement of one with respect to the other in the axial direction under the effect of a controlled deformation of at least one portion of said median or central part or segment,
said device (1) being **characterized in that** all the various parts or segments (5, 5', 6) of the hollow body (1') define respective chambers (7, 7', 6'),
the jaw-forming end part(s) or segment(s) (5, 5') comprise elastically deformable membranes as wall portions (8, 8') that are intended to come into contact with the elongate object (2) in a clamping phase, and
the chambers (7, 7') of the two end parts or segments (5, 5') are in fluidic communication with the chamber (6') of the median or central part or segment (6), each by way of at least one respective calibrated flow means (9, 9'), the injection of pressurized gaseous fluid taking place by way of a single feed line (10, 10') which is connected to a controlled source of pressurized fluid (11) and which opens into the chamber (7, 7') of one (5 or 5') of the two end parts or segments (5, 5'), depending on the direction of translational movement desired for the elongate object (2) present in the hollow body (1').

2. Device according to Claim 1, **characterized in that** the jaw-forming end parts or segments (5, 5'), respectively front and rear depending on the direction of positive movement of the elongate object (2), delimit chambers (7, 7') having an annular or rotationally toroidal shape about the axial direction (AD) and having a substantially non-deformable structure, axially and radially, within the range of operational pressures of fluid that is injected in normal operation of the device (1), with the exception of elastically deformable membranes forming internal sleeves (8, 8') that constitute the through-openings of the jaws for the elongate object (2), forming by alignment the axial passage (4) of the multi-chamber hollow body (1').

3. Device according to either of Claims 1 and 2, **characterized in that** the median or central part or segment (6) has at least one common wall portion (12, 12') with respectively each of the two end parts or segments (5, 5'), each of said common wall portions (12, 12') thus participating at the same time in the partial delimitation of one of the end chambers (7, 7') and **in that** of the median or central chamber (6'), and **in that** the calibrated flow means (9, 9') consist of through-holes which are formed in said common wall portions (12, 12') and which have defined shapes and sizes, depending on the desired sequencing for the inflation and the deflation of the chambers (7, 6', 7') determining the amplitude and/or the speed of the translational movement of the elongate object (2) in question.

4. Device according to any one of Claims 1 to 3, **characterized in that** the calibration of each of the respective flow means (9, 9') between each of the end chambers (5, 5') and the median or central chamber (6), which is optionally different for each means (9, 9'), is adjusted in such a way that, by passive monitoring of the circulation of the gaseous fluid injected via the single feed line (10, 10'), the two end chambers (5 and 5') can be under an operational pressure that brings about an effective double clamping of the object (2) that is to be moved, when the median or central chamber (6) is under operational pressure and in its deformed state.

5. Device according to any one of Claims 1 to 4, **characterized in that** the median or central part or segment (6) of the hollow body (1') is at least partially formed or covered by a layer (13) of an auxetic material, that is to say a material having a negative Poisson ratio.

6. Device according to any one of Claims 1 to 5, **characterized in that** the multi-chamber hollow body (1') with its three outputs or segments (5, 5') is made entirely of polymer material(s), preferably as an integrally formed one-part component, for example by three-dimensional printing, the deformable membranes (8, 8') of the jaws (5, 5') being optionally attached by overmoulding of a specific distinct polymer material.

7. Device according to any one of Claims 1 to 6, **characterized in that** a conduit (10, 10') that is able and intended to form selectively a feed line or a discharge line is connected to each of the chambers (7, 7') of the end parts or segments (5, 5').

8. Device according to any one of Claims 1 to 7, **characterized in that**
the median or central part or segment (6) defines an annular or toroidal chamber (6') and extends peripherally, and radially to the outside, about the two end parts or segments (5, 5') in order to give a multi-chamber hollow body (1') of disc-like shape, and **in that** it joins these parts or segments (5, 5') to each other by a connection elastically deformable in the axial direction (DA), the two end parts or segments (5, 5') being in mutual contact in said axial direction (DA) in the deflated state of the median or central part or segment (6), and the two end parts or segments (5, 5') being situated at a distance from each other in the inflated state of said median or central part or segment (6), under a pressure that is sufficient to bring about a deformation of the latter,
the median or central part or segment (6) of the multi-chamber hollow body (1') consists of, on the one hand, an internal tubular wall portion (14) that can be deformed elastically in the axial direction (DA) and connects the two end parts or segments (5, 5') to each other, and, on the other hand, of an outer wall portion of U-shaped cross section (15) that is of revolution about the axial direction (DA) and forms, by complementary cooperation with the internal tubular wall portion (14), the annular or toroidal median or central chamber (6') of the hollow body (1'), the two wings (15') of the U also being connected by their free ends to the two end parts or segments (5, 5') and being elastically deformable by outward bending of the U in the axial direction (DA), under the effect of a sufficient overpressure in the central or median chamber (6'), and
the zones (15") of the outer wall portion of U-shaped cross section (15), which correspond to the wings (15') of said U, are formed or covered by, preferably on their internal face, a layer (13) of auxetic material.

9. Device according to any one of Claims 1 to 7, **characterized in that** the median or central part or segment (6) comprises a cylindrical tubular chamber (6') extending in the axial direction (DA) and of revolution about the latter, said chamber (6') having an elastically deformable outer wall (16) and being in calibrated fluidic communication with the chambers (7, 7') of the end parts or segments (5, 5'), **in that** said wall (16) is covered on the outside by a cylindrical structure with an auxetic property (16'), which is formed by a latticed network with a repetitive pattern of elementary formations (17) and which bears on and is rigidly connected to two opposite disc-like stops (17'), which are also connected to the aforementioned deformable wall (16) and which form common wall portions (12, 12') with, or are connected to, the end parts or segments (5, 5'), the aforementioned constituent components of the median part or segment (6) being mutually arranged in such a way that the operational pressurization of the median or central chamber (6') brings about, by action of the wall (16) on the network (16'), a mutual spacing of the disc-like stops (17') in the axial direction (DA), and therefore of the end parts or segments (5, 5') that are respectively rigidly connected to said stops (17'), the elementary formations (17) together advantageously constituting a network (16') with an inverted honeycomb pattern.

10. Device according to any one of Claims 1 to 9, **characterized in that** the end parts or segments (5, 5') and the median or central part or segment (6) are aligned with one another and the elongate object (2) passes through all of them, the axial direction (DA) of the hollow body (1') and the axis of translation of the elongate object (2) being coincident.

11. Device according to any one of Claims 1 to 10, **characterized in that** the end parts or segments (5, 5') are aligned with each other and the elongate object (2) passes through both of them, the axis of translation of the elongate object (2) being coincident with the axis of alignment of said end parts or segments (5, 5') corresponding to the axial direction (DA), the median or central part or segment (6) being offset laterally or radially with respect to this direction (DA) and not being traversed by the elongate object (2).

12. Device according to any one of Claims 1 to 7, **characterized in that** the median or central part or segment (6) comprises three contiguous functional chambers or parts (22, 22', 23), namely, on the one hand, two chambers (22, 22') that are able and intended to ensure a function of movement under the effect of a deformation of at least one portion of their wall resulting from a modification of their internal pressure, and they are each coupled respectively to one of the inflatable jaw-forming end parts or segments (5, 5'), with which they are respectively in fluidic communication via a calibrated flow means (9, 9'), and, on the other hand, a jaw-forming median functional part or chamber (23), situated between the two afore-mentioned functional chambers or parts (22 and 22') and in fluidic communication with each of them via a respective calibrated flow means (24, 24'), all of the five parts (5, 5', 22, 22', 23) forming a cylindrical structure, a first of the two functional chambers (22) being advantageously configured to ensure a rotational movement about the axial direction (AD) of the elongate element (2) clamped by one (5) of the two end jaws (5, 5'), and the second of the two functional chambers (22') being advantageously configured to ensure a translational movement along the axial direction (AD) of the elongate object (2) clamped by the median jaw (23) and the other end jaw (5').

13. Device according to any one of Claims 1 to 12, **characterized in that** it comprises at least one adjustment means (25) for adjusting at least one calibrated flow means (9, 9', 24, 24'), in particular the passage section thereof, such an adjustment means (25) preferably being assigned to each flow means (9, 9', 24, 24'), the or each adjustment means (25), with manual or motorized actuation, advantageously comprising a movable sealing element for variable sealing of the calibrated orifice (9, 9', 24, 24') forming the flow means in question.

14. Device according to any one of Claims 1 to 13, **characterized in that** it comprises a sensor means (26), which is attached to or integrated in the median or central part or segment (6) and is able and intended to deliver a signal depending on the elongation of this part or of this segment in the axial direction (AD) and/or about the latter.

15. Device according to any one of Claims 1 to 14, **characterized in that** the elastically deformable internal membranes (8, 8', 23') of the jaw-forming parts or segments (5, 5', 23), defining a tube and coming into contact with the elongate object (2), have protruding formations (28) or surface textures ensuring a flexible centred hold of the elongate object (2) in the deflated and retracted state of said jaws, for example lips, the wall of at least one of the jaw-forming parts or segments (5, 5', 23), preferably the walls of all of these parts, advantageously comprising elastically deformable portions (29) in the inflated, active or clamped state of said jaw(s), providing an at least limited radial and/or axial structural compliance with respect to the elongate object (2) gripped by said jaw(s).

16. Device according to any one of Claims 1 to 15, **characterized in that** it comprises a solenoid valve (27) for controlling the injection of fluid, structurally coupled to one (5) of the jaw-forming end parts or segments (5, 5').

17. System for controlled sequential holding and moving of an elongate object, such as a surgical or medical instrument or tool of oblong shape, intended to be introduced into the body of a patient,
said system comprising, on the one hand, at least one pneumatic holding and moving device carried by a specific support or rigidly attached to the patient, and placed in direct contact with the skin of the latter, and, on the other hand, at least one source of pressurized gaseous fluid, and, finally, a human-machine control interface, optionally forming part of a medical installation, said system being **characterized in that** said at least one pneumatic holding and moving device consists of a device (1) according to any one of Claims 1 to 16, its hollow body (1') being held at its front end part (5).

18. Controlled and preferably automated system for insertion and optionally assembly or mounting, by translational movement, optionally combined with a rotational movement, of an elongate object, such as a structural component, tool or instrument, in a suitable passage, opening or receiving seat of another object, component, part, element or the like,
said system being **characterized in that** it comprises at least one guided pneumatic holding and moving device (1) according to any one of Claims 1 to 16, said device (1) being mounted on an optionally movable support means, such as a robot arm or a collaborative robot, said device (1) forming, if appropriate, an integral part of said robot arm or robot.
